# EUROPEAN PATENT APPLICATION

(11) **EP 4 738 318 A1**
(43) Date of publication of application: **06.05.2026**
(21) Application number: 24849682.0
(22) Date of filing: 26.07.2024
(51) Int. Cl.: G09B 21/00, G09B 5/06, G10L 21/10, G06F 3/041, G06F 3/0488, G06F 3/0484, G06F 3/0482, G06F 3/04817, G09B 7/02, G06Q 50/10

(54) **AUDITORY TRAINING METHOD AND DEVICE**

(30) Priority: 28.07.2023 KR 20230098992; 19.02.2024 KR 20240023586; 25.07.2024 KR 20240098856
(71) Applicant: Bell Therapeutics Inc., Seoul 08735 (KR)
(72) Inventor: PARK, Jong Hwa, Seoul 06255 (KR); PARK, Jeong Mi, Suwon-si, Gyeonggi-do 16547 (KR); LEE, Won Woo, Goyang-si, Gyeonggi-do 10362 (KR); HA, Ji Yeon, Yangju-si, Gyeonggi-do 11488 (KR); LEE, Jae Eun, Seoul 06543 (KR); KIM, Jung Hoo, Seoul 06631 (KR); YUM, Joong Bae, Seoul 04747 (KR)
(74) Representative: HGF
(86) International application number: PCT/KR2024/095938
(87) International publication number: WO 2025/029115

(57) **Abstract**

An auditory training method, according to one embodiment, may comprise an operation of providing a test sound having a first auditory pattern for at least one feature of sound. The auditory training method may comprise an operation of providing, through a user interface for receiving a visual pattern, a first visual object having a first visual pattern corresponding to a user input on the basis of the user input that is input through the user interface. A first sound which has at least one feature identified on the basis of a location defined in the user interface of the user input and which is substantially synchronized with a detection point of the user input can be provided. The auditory training method may comprise an operation of providing a second visual object having a second visual pattern corresponding to at least one feature of the first auditory pattern of the test sound on the basis of event identification for providing the second visual pattern corresponding to the test sound.

## Description

### [Technical Field]

The present invention relates to a method and apparatus for auditory training.

### [Background Art]

As the population ages, the number of individuals with hearing impairments due to presbycusis is increasing. Hearing specialists predict that, with the rise in average life expectancy, the number of individuals with hearing impairments will further increase. Additionally, hearing impairments can occur at any age due to congenital or acquired causes.

Accordingly, there is growing interest in assistive devices for individuals with hearing impairments (e.g., hearing aids and cochlear implants). In particular, there is increasing interest in cochlear implants for individuals with severe hearing loss who do not experience significant improvements in hearing even with the use of hearing aids. However, even after cochlear implant surgery, rehabilitation training is essential as the ability to hear sounds is not immediate.

Currently known rehabilitation training methods mainly involve listening repeatedly to recorded sounds (e.g., words, short sentences) and solving problems based on them. These methods are monotonous and inefficient, as they primarily rely on repetitive and uniform exercises, which may lead to user boredom. Meanwhile, even individuals who do not experience discomfort in daily life due to hearing issues may seek to improve their auditory functions for various reasons (e.g., enhancing musical abilities, developing talents in infants and young children).

Therefore, there is a need for the development of user-friendly and effective auditory training (or auditory rehabilitation training) methods that take into account the individual characteristics of users, including those with hearing loss and/or those seeking to enhance their auditory functions.

Currently known rehabilitation training methods may include providing sounds corresponding to words or short sentences with semantic meaning (syntax) repeatedly and evaluating whether the user has correctly perceived the sounds based on user input corresponding to these sounds.

### [Description of the Invention]

### [Problems to be Solved]

According to conventional auditory training methods, sounds corresponding to words or short sentences are provided, enabling individuals with hearing loss to listen to the sounds and check whether their auditory recognition results match the correct answers. However, there are cases where the auditory recognition results of individuals with hearing loss differ from the actual sounds. In such cases, while individuals with hearing loss may realize that their recognition results are different from the actual sounds, this realization alone may lead to prolonged rehabilitation periods. Individuals with hearing loss need to engage in extended training sessions to listen to sounds repeatedly in order to reduce the discrepancy between the sounds they perceive and the actual sounds. As a result, the process of auditory rehabilitation can be time-consuming. Therefore, there is a demand for technologies that visually provide the characteristics of actual sounds along with the actual sounds themselves and/or receive input from individuals with hearing loss regarding the characteristics of the sounds.

Methods that provide sounds corresponding to words or short sentences are limited in their ability to provide sounds covering a wide range of frequency bands. Many individuals with hearing loss struggle to perceive sounds in specific frequency bands. However, when conventional sounds corresponding to meaningful words or short sentences are provided, it may not be possible to deliver sounds across a wide frequency range and/or sounds within the frequency bands where the individual with hearing loss has difficulty may not be provided. Accordingly, there is a need for the development of technologies that provide not only sounds corresponding to words or short sentences but also sounds corresponding to various frequencies and/or targeted frequency bands.

The technical problems addressed by the present invention are not limited to the aforementioned issues, and additional technical problems not explicitly mentioned will be readily understood by those skilled in the art from the following descriptions.

### [Means to Solve the Problem]

According to one embodiment, a method for auditory training may include: providing a test sound having a first auditory pattern for at least one characteristic of a sound; providing, through a user interface for receiving a visual pattern, a first visual object having a first visual pattern corresponding to a user input that is input through the user interface; and providing a second visual object having a second visual pattern corresponding to at least one characteristic of the first auditory pattern of the test sound, based on identifying an event for providing the second visual pattern corresponding to the test sound. A first sound, which is substantially synchronized with a detection time point of the user input and has at least one characteristic identified based on the position of the user input defined in the user interface, may be provided.

According to one embodiment, providing the first visual object through the user interface may include: based on detecting at least a part of the user input associated with a first position and a second position of the user interface, providing at least a part of the first visual object associated with the first position, the second position, and at least one intermediate position between the first and second positions of the user interface.

According to one embodiment, based on at least a part of the user input being associated with the first position, a first portion of the first sound having at least one characteristic corresponding to the first position may be provided; based on at least a part of the user input being associated with each of the at least one intermediate position, at least one intermediate portion of the first sound having at least one characteristic corresponding to each intermediate position may be provided; and based on at least a part of the user input being associated with the second position, a second portion of the first sound having at least one characteristic corresponding to the second position may be provided.

According to one embodiment, at least a part of the user input may include an input for designating the first position and the second position, and/or an input for designating the first position, the at least one intermediate position, and the second position.

According to one embodiment, the test sound may include a plurality of portions provided sequentially over time, and each of the plurality of portions may have at least one characteristic that changes or remains constant over time according to the first auditory pattern.

According to one embodiment, the method may further include providing a result of comparing the first visual object and the second visual object.

According to another embodiment, the operation of providing the comparison result may include providing information about a user's vulnerable points identified based on the comparison result.

According to one embodiment, the event for providing the second visual pattern corresponding to the test sound may include a selection for affordance to provide a correct visual object, completion of providing the first visual object, and/or passage of a specified time.

According to another embodiment, the method may further include performing an operation of modifying at least a part of the first visual object based on another user input for modifying the first visual object.

According to one embodiment, the operation of modifying at least a part of the first visual object may include identifying a deletion command for a first portion of the first visual object and deleting the first portion associated with the deletion command while maintaining the display of the remaining portion of the first visual object excluding the first portion.

According to another embodiment, at a certain time, each of at least one characteristic of at least a portion of the test sound may have a single value.

In another embodiment, at a certain time, each of at least one characteristic of at least a portion of the test sound may have multiple values.

According to one embodiment, the user interface may include a plurality of reference objects for association with the user input.

In another embodiment, the plurality of reference objects may be arranged in a grid, and the user input may include an input connecting one of a plurality of first reference objects included in one column to one of a plurality of second reference objects included in an adjacent column.

According to one embodiment, based on two or more reference objects being associated with a first temporary user input, a visual object associated with the two or more reference objects may be provided as the first visual object; and based on two or more reference objects not being associated with a second temporary user input, the display of the visual object temporarily provided based on the trajectory of the second temporary user input may be discontinued.

According to another embodiment, the number, density, and/or arrangement of the plurality of reference objects may be set based on user selection and/or test difficulty.

In one embodiment, the lower limit value, upper limit value, and/or the difference between the upper and lower limit values of the test sound may be set based on user selection and/or test difficulty.

According to another embodiment, sound effects identified based on user selection and/or test difficulty may be applied to at least a portion of the test sound.

In one embodiment, background sound identified based on user selection and/or test difficulty may be provided along with at least a portion of the test sound.

According to another embodiment, the method may further include setting at least one different characteristic of a sound other than the at least one characteristic of the first auditory pattern based on user selection and/or test difficulty.

In one embodiment, the method may further include providing at least one indicator visually notifying the playback point of the test sound over time along with the provision of the test sound.

According to one embodiment, a system for auditory training may include a server and an electronic device including at least one processor. The server may be configured to provide instructions to the electronic device based on a connection to the server by the electronic device and/or a request to the server. The instructions, when executed based on at least a portion of the at least one processor of the electronic device, may cause the electronic device to perform at least one operation, and the at least one operation may include providing a test sound having a first auditory pattern for at least one characteristic of a sound, providing, through a user interface for receiving a visual pattern, a first visual object having a first visual pattern corresponding to a user input that is input through the user interface, and providing a second visual object having a second visual pattern corresponding to at least one characteristic of the first auditory pattern of the test sound. A first sound, which is substantially synchronized with a detection time point of the user input and has at least one characteristic identified based on the position of the user input defined in the user interface, may be provided.

According to one embodiment, a method for auditory training by a system including a server and an electronic device may include providing, by the server, instructions to the electronic device based on a connection to the server by the electronic device and/or a request to the server. The method may include executing, by the electronic device, the instructions, wherein the instructions, when executed based on at least a portion of at least one processor of the electronic device, cause the electronic device to perform at least one operation, and the at least one operation may include providing a test sound having a first auditory pattern for at least one characteristic of a sound, providing, through a user interface for receiving a visual pattern, a first visual object having a first visual pattern corresponding to a user input that is input through the user interface, and providing a second visual object having a second visual pattern corresponding to at least one characteristic of the first auditory pattern of the test sound, based on identifying an event for providing the second visual pattern corresponding to the test sound. A first sound, which is substantially synchronized with a detection time point of the user input and has at least one characteristic identified based on the position of the user input defined in the user interface, may be provided.

According to one embodiment, a method for auditory training by a system including a server and an electronic device may include providing a server having at least one first processor. The server may be configured to provide instructions to the electronic device based on a connection to the server by the electronic device and/or a request to the server. The method may include executing, by the electronic device, the instructions, wherein the instructions, when executed based on at least a portion of at least one processor of the electronic device, cause the electronic device to perform at least one operation. The at least one operation may include providing a test sound having a first auditory pattern for at least one characteristic of a sound, providing, through a user interface for receiving a visual pattern, a first visual object having a first visual pattern corresponding to a user input that is input through the user interface, and providing a second visual object having a second visual pattern corresponding to at least one characteristic of the first auditory pattern of the test sound, based on identifying an event for providing the second visual pattern corresponding to the test sound. A first sound, which is substantially synchronized with a detection time point of the user input and has at least one characteristic identified based on the position of the user input defined in the user interface, may be provided.

According to one embodiment, a storage medium storing computer-readable instructions may include instructions that, when executed by at least one processor of an electronic device, cause the electronic device to perform at least one operation. The at least one operation may include providing a test sound having a first auditory pattern for at least one characteristic of a sound, providing, through a user interface for receiving a visual pattern, a first visual object having a first visual pattern corresponding to a user input that is input through the user interface, and providing a second visual object having a second visual pattern corresponding to at least one characteristic of the first auditory pattern of the test sound, based on identifying an event for providing the second visual pattern corresponding to the test sound. A first sound, which is substantially synchronized with a detection time point of the user input and has at least one characteristic identified based on the position of the user input defined in the user interface, may be provided.

According to one embodiment, an electronic device may include at least one processor and a memory storing instructions. The instructions, when executed based on at least a portion of the at least one processor, may cause the electronic device to perform at least one operation. The at least one operation may include providing a test sound having a first auditory pattern for at least one characteristic of a sound, providing, through a user interface for receiving a visual pattern, a first visual object having a first visual pattern corresponding to a user input that is input through the user interface, and providing a second visual object having a second visual pattern corresponding to at least one characteristic of the first auditory pattern of the test sound, based on identifying an event for providing the second visual pattern corresponding to the test sound. A first sound, which is substantially synchronized with a detection time point of the user input and has at least one characteristic identified based on the position of the user input defined in the user interface, may be provided.

### [Effects of the Invention]

According to various embodiments, implementations may be provided in which the characteristics of a sound are visually presented along with auditory training sounds for individuals with hearing loss, and/or the characteristics of the sound are received as input from individuals with hearing loss.

According to various embodiments, sounds corresponding not only to words or short sentences but also to various frequencies and/or targeted frequencies may be provided.

According to various embodiments, not only language-based sounds with semantic meaning but also non-language-based sounds without semantic meaning may be provided.

According to various embodiments, sound characteristics to which individuals with hearing loss are vulnerable may be identified, allowing sounds with the identified characteristics to be provided.

The effects of the present invention are not limited to those mentioned above, and additional effects not explicitly mentioned will be readily understood by those skilled in the art based on the claims and/or the detailed description of the invention.

### [Brief Description of the Drawings]

FIG. 1a is a diagram illustrating an electronic device, a server, and an external electronic device according to an embodiment of the present invention.
FIG. 1b is a diagram illustrating a system for providing a service according to an embodiment.
FIG. 1c is a diagram illustrating a method for providing a service according to an embodiment.
FIG. 2 is a flowchart illustrating a method for providing auditory training content according to an embodiment of the present invention.
FIGS. 3a to 3c are examples of screens provided by an electronic device according to various embodiments.
FIG. 4 is a diagram illustrating a method for providing auditory training content according to an embodiment.
FIGS. 5a to 5i are diagrams illustrating content provided by an electronic device according to various embodiments.
FIG. 6a is a diagram illustrating a method for providing auditory training content according to an embodiment.
FIG. 6b is a diagram illustrating a method for providing auditory training content according to an embodiment.
FIG. 7 is a diagram illustrating a method for providing auditory training content according to an embodiment.
FIG. 8 is a diagram illustrating a method for providing auditory training content according to an embodiment.
FIG. 9 is a diagram illustrating a method for providing auditory training content according to an embodiment.
FIG. 10a is a diagram illustrating a method for providing auditory training content according to an embodiment.
FIG. 10b is a diagram illustrating multiple sounds according to an embodiment.
FIG. 11a is a diagram illustrating a method for providing auditory training content according to an embodiment.
FIG. 11b is a diagram illustrating a user interface according to an embodiment.
FIG. 12a is a diagram illustrating a method for providing auditory training content according to an embodiment.
FIG. 12b is a diagram illustrating user interfaces according to different levels of test difficulty.
FIG. 12c is a diagram illustrating a method for providing auditory training content according to an embodiment.
FIG. 12d is a diagram illustrating user interfaces according to different levels of test difficulty.
FIG. 12e is a diagram illustrating a method for providing auditory training content according to an embodiment.
FIG. 12f is a diagram illustrating a method for providing auditory training content according to an embodiment.
FIG. 12g is a diagram illustrating the application of sound effects according to an embodiment.
FIG. 12h is a diagram illustrating a method for providing auditory training content according to an embodiment.
FIG. 13a is a diagram illustrating a method for providing auditory training content according to an embodiment.
FIG. 13b is a diagram illustrating the position of a virtual sound source in a stereo environment according to an embodiment.
FIGS. 13c to 13f are diagrams illustrating screens for providing auditory training content according to various embodiments.
FIG. 14a is a diagram illustrating a method for providing auditory training content according to an embodiment.
FIG. 14b is a diagram illustrating analysis based on formants according to an embodiment.
FIG. 15 is a diagram illustrating auditory training content according to an embodiment.
FIGS. 16a to 16i are diagrams illustrating auditory training content according to various embodiments.
FIG. 17 is a diagram illustrating auditory training content according to an embodiment.
FIGS. 18a and 18b are diagrams illustrating auditory training content according to various embodiments.
FIG. 19 is a diagram illustrating changes in auditory evaluation resulting from performing auditory training content according to an embodiment.

### [Detailed Description of the Invention]

Hereinafter, preferred embodiments of the present invention will be described in detail with reference to the accompanying drawings. The advantages, features, and methods for achieving these will become apparent by referring to the embodiments described in detail below along with the attached drawings. However, the present invention is not limited to the embodiments disclosed below and may be implemented in various other forms. The embodiments are provided to fully disclose the present invention and inform those skilled in the art of the scope of the invention, which is defined only by the claims. Identical reference numerals in the following description denote identical components.

Although terms such as "first," "second," etc., are used to describe various elements, components, and/or sections, these terms are not intended to limit these elements, components, and/or sections. These terms are only used to distinguish one element, component, or section from another. Accordingly, a "first" element, component, or section mentioned in the following may also be a "second" element, component, or section within the technical spirit of the present invention.

The terms used in this specification are for describing the embodiments and are not intended to limit the present invention. Unless explicitly stated otherwise, the singular forms used herein include the plural as well. The terms "comprises" and/or "made of," as used herein, do not exclude the presence or addition of one or more other elements, steps, operations, and/or components.

In this specification, when a part is said to be "connected" to another part, it may refer not only to physical contact (or fastening) but also to being connected through an intermediate entity. Accordingly, "connection" may refer to a state of direct physical contact or a state where another entity is interposed. Furthermore, "connection" may refer to not only a physical connection but also a "logical connection," which may imply connection based on wireless communication.

The identification codes in the steps are used for convenience in explanation and do not describe the order of the steps. Unless a specific order is explicitly stated in the context, the steps may be performed in an order different from that specified.

Unless otherwise defined, all terms used herein (including technical and scientific terms) can be understood as having meanings commonly understood by those skilled in the art to which the invention pertains. Additionally, terms defined in general dictionaries should not be interpreted ideally or excessively unless explicitly defined otherwise.

The various embodiments described in this specification may be implemented as software (e.g., programs) including one or more instructions stored in a machine-readable storage medium (e.g., internal or external memory). For example, a processor of a machine (e.g., an electronic device) may retrieve at least one instruction from the storage medium and execute it. This allows the machine to perform at least one function according to the retrieved instruction(s). The instruction(s) may include code generated by a compiler or code executable by an interpreter. The storage medium readable by the machine may be provided in the form of a non-transitory storage medium. Here, "non-transitory" means the storage medium is a tangible device and does not include signals (e.g., electromagnetic waves), but this term does not distinguish whether data is stored on the storage medium permanently or temporarily.

Methods according to various embodiments of this specification may be provided in a computer program product. The computer program product may be traded as a commodity between sellers and buyers. It may be distributed in the form of a machine-readable storage medium (e.g., compact disc read-only memory (CD-ROM)) or distributed online (e.g., downloaded or uploaded) through an application store (e.g., Play Store^{™}) or directly between two user devices (e.g., smartphones). In the case of online distribution, at least a portion of the computer program product may be temporarily stored or generated in a machine-readable storage medium, such as the memory of a manufacturer's server, an application store server, or an intermediary server.

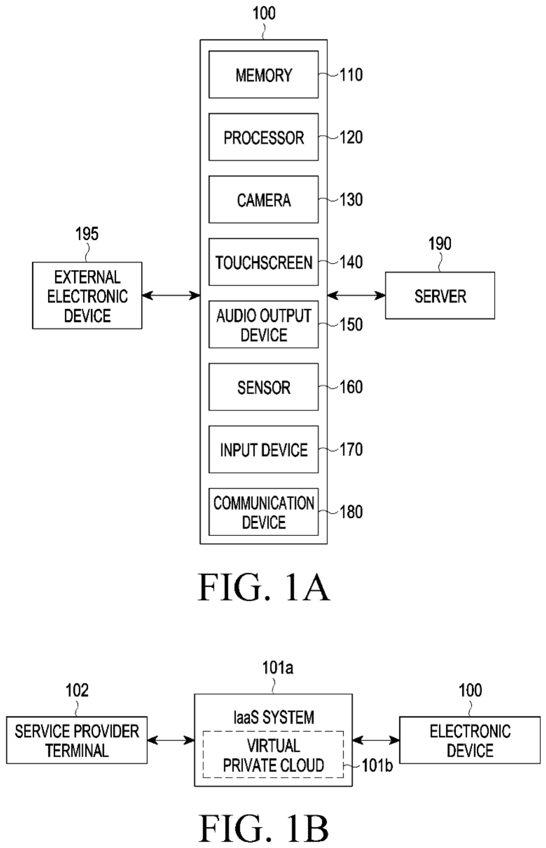

FIG. 1a is a diagram illustrating an electronic device, a server, and an external electronic device according to an embodiment of the present invention.

According to one embodiment, the electronic device 100 may be implemented in the form of a portable electronic device (e.g., a smartphone or tablet PC, but not limited thereto), a wearable electronic device (e.g., a watch, ring, bracelet, anklet, necklace, glasses, contact lenses, or head-mounted device (HMD), but not limited thereto), or a computer. However, those skilled in the art will understand that the electronic device 100 is not limited to these forms as long as it has the capability to provide visual, auditory, and/or tactile content. In one example, the electronic device 100 may perform at least some of the operations conducted according to various embodiments of the present disclosure without integration with other external electronic devices (this can be referred to as a stand-alone type). For example, the electronic device 100 may store an application for stand-alone operation and perform at least some operations according to the various embodiments of the present disclosure by executing the stored application. In another example, the electronic device 100 may perform at least some of the operations conducted according to various embodiments of the present disclosure in conjunction with other external electronic devices. For instance, the electronic device 100 may provide data to, or receive data from, a server (e.g., a web server, application server, computing server, database server, file server, game server, mail server, proxy server, and/or cloud server, but not limited thereto) 190. Based on this integration, the electronic device 100 may execute at least some of the operations conducted according to the various embodiments of the present disclosure. For example, the electronic device 100 may temporarily or permanently store a program (or, alternatively referred to as at least one instruction or algorithm, but not limited thereto) provided by the server 190. As the program is executed by the electronic device 100, at least some of the operations described in the present disclosure may be performed. For instance, a web server may provide data for representing content according to various embodiments of the present disclosure based on a request from the electronic device 100. The electronic device 100 may make requests based on a web browsing application or a PWA (progressive web application), but the request method is not limited. The web server may independently acquire data for representing content and/or generate data based on information provided by an additional database server. However, those skilled in the art will understand that the method for generating such data is not restricted. Alternatively, the electronic device 100 may download and install an application capable of providing the operations described in the present disclosure from an application marketplace. By executing the installed application, the electronic device 100 may provide the operations described in the present disclosure. Moreover, those skilled in the art will understand that an application for providing the operations described in the present disclosure may be pre-installed on the electronic device 100.

Referring to FIG. 1a, the electronic device 100 according to an embodiment of the present invention can provide content for auditory training to users who wish to enhance their auditory functions. For example, the electronic device 100 of the present invention may provide auditory training (or rehabilitation training) content for users such as individuals with hearing impairments wearing cochlear implants and users seeking to improve their auditory functions for various reasons (e.g., elderly individuals, infants, or musicians).

According to one embodiment, the electronic device 100 may include a memory 110, a processor 120, a camera 130, a touchscreen 140, an audio output device 150, sensors 160, an input device 170, and/or a communication device 180.

The memory 110 may include storage media of at least one type, such as a flash memory type, hard disk type, SSD type (Solid State Disk type), SDD type (Silicon Disk Drive type), multimedia card micro type, card-type memory (e.g., SD or XD memory), RAM (random access memory), SRAM (static random access memory), ROM (read-only memory), EEPROM (electrically erasable programmable read-only memory), PROM (programmable read-only memory), magnetic memory, magnetic disks, or optical disks. Those skilled in the art will understand that the memory 110 may also refer to, for example, cache memory for interfacing with the processor 120 and/or cache memory and/or registers included within the processor 120.

The memory 110 may permanently or temporarily store a program (or application) that causes the execution of at least some of the operations performed by the electronic device 100 as disclosed herein. Those skilled in the art will understand that the program (or the instructions constituting the program) may be stored in a single entity or distributed across multiple entities. The electronic device 100 may download an application (e.g., from an application marketplace or an application source) and store it in the memory 110. By executing the application, the electronic device 100 can perform operations conducted according to various embodiments of the present disclosure. Alternatively, the electronic device 100 may download data from the server 190, which causes the electronic device to perform operations conducted according to various embodiments of the present disclosure, and store this data in the memory 110. For example, the electronic device 100 may temporarily store data in the memory 110 and use it to provide content for auditory training. Subsequently, the device may either delete the data or retain it.

The processor 120 may include a CPU, GPU, NPU, DPU, FPGA, ASIC, and/or SoC, and its implementation is not limited to a specific form. For example, according to certain embodiments, an operation performed by the electronic device 100 and/or the server may be executed by any one of the processors (e.g., CPU, GPU, NPU, FPGA, ASIC, and/or SoC) or through the collaboration of two or more processors. For instance, multiple operations performed by the electronic device 100 and/or the server 190 may be executed by any one of the processors (e.g., CPU, GPU, NPU, FPGA, ASIC, and/or SoC) or distributed such that some operations are executed by one processor while others are performed by another processor. The electronic device 100 and/or server may include at least one memory for storing at least one instruction. The memory may include volatile memory and/or non-volatile memory, without limitation to specific types. When executed by at least one processor 120, the at least one instruction may cause the electronic device 100 and/or the server 190 to perform at least one operation (e.g., at least some of the operations described in this disclosure). The instruction(s) causing the execution of one or more operations by the electronic device 100 and/or server 190 may be stored in a single physically independent memory or distributed across multiple memories. Additionally, at least one processor may operate based on inference from at least one artificial intelligence (AI) model. The AI model may be trained using training data. Such training can be performed on the device implementing AI according to this disclosure or through a separate server and/or system. Examples of training algorithms include supervised learning, unsupervised learning, semi-supervised learning, or reinforcement learning, but are not limited to these. The processor may also operate based on designated rule-based logic.

The camera 130 can capture external scenes and provide images corresponding to the external scenes. For example, the camera 130 may be activated to capture external scenes during the execution of an application for auditory training (e.g., when the interface screen is displayed), although the activation conditions are not limited. Those skilled in the art will understand that the camera 130 may be implemented as one or multiple units. The processor 120 may analyze at least one image provided by the camera 130 to identify motion input based on a part of the user's body included in the image. For instance, at least a portion of one or more sub-user inputs detected by the electronic device 100, as described later, may be identified based on images captured by the camera 130. For example, if the electronic device 100 is implemented as a wearable electronic device, such as an HMD or glasses, user input may be identified (or recognized) based on the analysis of images provided by the camera 130 for hand tracking.

The touchscreen 140 may include a display for performing display functions and a touch panel for performing input functions. For example, the touchscreen 140 can output various screens for auditory training (e.g., interface screens, visual data, etc.). Additionally, the touchscreen 140 can detect user touch input based on the interface. On the other hand, those skilled in the art will understand that if the electronic device 100 is implemented in a form other than a smartphone, the touchscreen 140 may be replaced by another entity. For example, if the electronic device 100 is implemented as an HMD, it may include a display device for displaying screens. In another example, if the electronic device 100 is implemented as a glasses-type wearable electronic device, it may include a projector, an optical waveguide, and/or lenses for displaying screens.

The audio output device 150 can output sound. According to one embodiment, the processor 120 may control the audio output device 150 to output sound in real-time based on user input detected through the interface screen. For instance, the processor 120 can provide sound composed of segments corresponding to multiple sub-user inputs. The attributes of each sound segment corresponding to the sub-user inputs (e.g., frequency, overtone density, timbre, and/or volume, but not limited to these) may be determined based on the input characteristics of each sub-user input (e.g., input type or property, but not limited to these) and/or input position. The attributes of adjacent segments of the sound may differ, or they may be the same. The provision of sound segments corresponding to the input (or detection, identification, or maintenance) of sub-user inputs may temporally overlap, but the implementation is not limited. Additionally, the processor 120 may control the audio output device 150 to provide test sounds for auditory training before receiving user input. Meanwhile, the electronic device 100 may also provide data for sound output to an external audio output device (not shown) connected to the electronic device 100 via a wired or wireless connection.

The sensor 160 may include at least one of the following: a proximity sensor, an illumination sensor, a touch sensor (which may be implemented as part of the touchscreen 140 or as separate hardware, with no limitations on its implementation), an acceleration sensor, a magnetic sensor, a gravity sensor (G-sensor), a gyroscope sensor, a motion sensor, an RGB sensor, an infrared sensor (IR sensor), a fingerprint recognition sensor, an ultrasonic sensor, an optical sensor (e.g., a camera), a microphone, environmental sensors (e.g., barometer, hygrometer, thermometer, radiation detection sensor, thermal detection sensor, gas detection sensor), or biometric sensors (e.g., healthcare sensors, biometric recognition sensors). Moreover, the device may combine and utilize information sensed by at least two or more of these sensors.

The input device 170 may be configured to receive user input. The input device 170 may be implemented as, for example, a button, a touchpad, or a touch sensor as part of the touchscreen 140, without limitations on its implementation. The electronic device 100 can identify user input through the input device 170. Additionally, the electronic device 100 may identify user input based on data received from an external electronic device 195 connected via wired or wireless communication, rather than through the input device 170 included in the electronic device 100. For instance, the external electronic device 195 may include sensors and/or input devices for sensing user input and provide data required for user input identification to the electronic device 100. The electronic device 100 can identify user input based on data provided by the external electronic device 195. The external electronic device 195 may, for example, be implemented as a user controller for an HMD; however, this is merely illustrative, and the implementation is not limited to this example.

The communication device 180 may include one or more components that enable communication with various devices equipped with communication capabilities. For example, it may include at least one of the following: a wired communication device, a cellular-based wireless communication device, an IEEE 802.11-based wireless communication device (e.g., commonly referred to as Wi-Fi), a short-range communication device (e.g., Bluetooth, Bluetooth Low Energy, UWB, Zigbee, but not limited to these), or a location information module. For instance, the electronic device 100 can transmit and/or receive data to and from the server 190 via the communication device 180. The server 190 may provide data to the electronic device 100 that causes the execution of at least some of the operations conducted according to various embodiments of this disclosure. For example, those skilled in the art will understand that if the electronic device 100 is implemented as a stand-alone type, data transmission and/or reception between the electronic device 100 and the server 190 may not be required. The communication device 180 may include components such as a transceiver, a communicator, and so on.

FIG. 1b is a diagram illustrating a system for providing services according to an embodiment.

According to one embodiment, the service providing terminal 102 may provide data for creating a virtual private cloud (VPC) 101b to an IaaS (Infrastructure as a Service) system 101a. The IaaS system 101a may provide hardware resources for executing an operating system and/or programs based on client requests. For example, based on a request from the service providing terminal 102, the IaaS system 101a may execute and/or create the virtual private cloud 101b for providing services (e.g., content for auditory training, but not limited to this). The virtual private cloud 101b may provide data to the electronic device 100, which is the user terminal described in this disclosure, to cause the execution of operations performed by the electronic device 100. The electronic device 100 may perform at least one operation to access the virtual private cloud 101b. Based on the access procedure, the virtual private cloud 101b may provide data to the electronic device 100 to cause the execution of operations performed by the user terminal, as described in this disclosure.

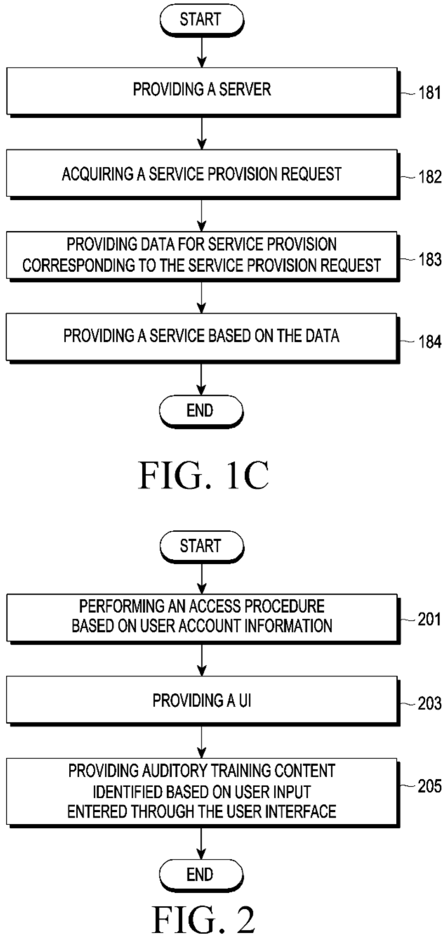

FIG. 1c is a diagram illustrating a method for providing services according to an embodiment.

According to one embodiment, the method may include an operation 181 of providing a server. The operation 181 of providing a server may include, for example, creating, constructing, and/or activating a physical device, such as the server 101, although it is not limited to these. Alternatively, the operation 181 of providing a server may include transmitting a request for creating a virtual private cloud (VPC) 101b, as described in FIG. 1b, as well as the creation, execution, and/or activation of the VPC 101b within the IaaS system 101a, without limitation.

According to one embodiment, the method may include an operation 182 of acquiring a service provision request. The method may also include an operation 183 of providing data for service provision corresponding to the service provision request. For example, the electronic device 100 may perform at least one operation to access the server 101 (or virtual private cloud 101b). The server 101 (or virtual private cloud 101b) may interpret the at least one access operation as an acquisition of a service provision request and provide data for service provision to the electronic device 100. For instance, after performing at least one access operation, the server 101 (or virtual private cloud 101b) may also be implemented to receive additional service provision requests from the electronic device 100. Based on the receipt of a service provision request, the server 101 (or virtual private cloud 101b) can provide data for service provision to the electronic device 100.

According to an embodiment, the method may include an operation 184 of providing a service based on the data. For example, the electronic device 100 may provide the service described in this disclosure based on data received from the server 101 (or virtual private cloud 101b). Details regarding the provided service will be described later. The electronic device 100 may temporarily store the received data for service provision and subsequently delete it. Alternatively, the electronic device 100 may store the received data and delete it upon identifying an additional delete command. For instance, the electronic device 100 may provide services using a cloud-based approach or by downloading and executing an application package. The method of service provision is not limited. If the application package is downloaded, the server 101 may be implemented as the source of the application package. However, this is merely illustrative, as an application marketplace (not shown) may also serve as the source of the application package. The service provider terminal 102 may generate an application package associated with instructions for causing the operations described in this disclosure and/or provide it to a source (e.g., a server and/or an application marketplace).

FIG. 2 is a flowchart illustrating a method for providing auditory training content according to an embodiment of the present invention.

Referring to FIG. 2, the method according to one embodiment may include an operation 201 of performing an access procedure based on user account information. For example, the electronic device 100 may perform an access procedure to the server 190 (e.g., accessing a web page via an internet browser or executing a pre-installed application, without limitation). The access procedure may include, for example, a login process for the auditory training service by entering pre-registered account information (e.g., ID and/or password, or biometric information such as fingerprints, iris scans, facial images, etc.). However, this is illustrative, and the login process may also be performed using an auto-login feature. For instance, the method may include an operation of verifying user information, although this is not mandatory. User information may include, for example, auditory training details (e.g., auditory training services completed by the user, daily training progress, weekly training progress, monthly training progress, etc.), but it is not limited to these. The auditory training content may be configured dependent on the user information. However, those skilled in the art will understand that the content can also be configured independently of the user information.

The method may include an operation 203 of providing a user interface (UI). The user interface may be configured, for example, to receive user input for providing auditory training content and/or to deliver content based on the user input. Details on various examples of this will be described later. The method may provide auditory training content identified based on user input entered through the user interface. The auditory training content may include, for instance, non-linguistic training content and linguistic training content, though the criteria for content classification are not limited. For example, non-linguistic training content may include sound picture watching content or sound drawing content, but it is not restricted to these. Detailed explanations will be provided later. Similarly, linguistic training services may include phoneme training content, although this is not limiting. Phoneme training content may include, for example, phoneme familiarization content, phoneme pair training content, or labeling training content, though it is not limited to these. Further details will be described subsequently.

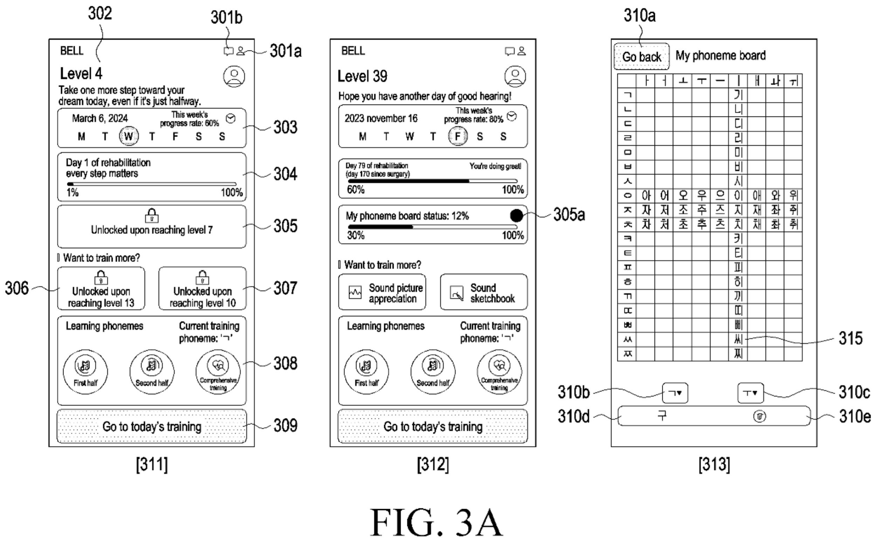

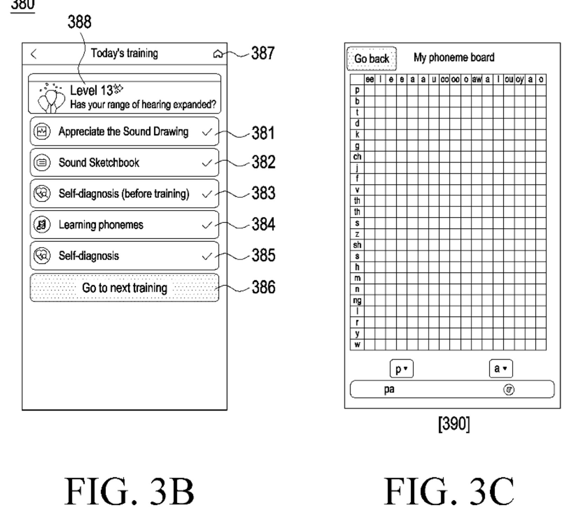

FIGs. 3a and 3b illustrate examples of screens provided by an electronic device according to embodiments.

According to one embodiment, the electronic device 100 may display a first screen 311, as shown in FIG. 3a. The first screen 311 may, for instance, be provided after accessing the auditory training service, although the sequence of provision is not limited. For example, the first screen 311 may include an object (or visual element, affordance, icon, button, popup window, etc.) 301a related to user profile information. Based on the designation of the object 301a, the electronic device 100 may provide user profile information or enable the modification of user profile information. User profile information may include personal details, training service start information, cochlear implant surgery-related information, current level information, and usage instructions, though it is not limited to these. The first screen 311 may also include an object 301b for providing a chat service with an administrator. Based on the selection of object 301b, the electronic device 100 may provide a chat service (e.g., displaying a chat window), without limitation.

For example, the first screen 311 may include user level information 302. The user level information 302 may indicate the user's current training level, which could be determined based on the repetition frequency of training services. Alternatively, user level may be determined based on evaluations of training performance. Depending on the user's training level, the difficulty of the content, the types of accessible content, and/or content configuration settings may be determined, but these are not limiting.

The first screen 311 may also include information 303 related to progress. This progress-related information 303 may include a weekly calendar and a weekly progress rate (e.g., the ratio of training completed by the user compared to the total training required for the week). Additionally, the first screen 311 may include training cycle information 304, representing the training period. For example, training cycle information 304 may be displayed as a numerical representation of the training period and/or a bar graph indicating the progress compared to the set period, without limitation. The first screen 311 may also display phoneme chart status information 305 in a deactivated state. As shown in FIG. 3a, some areas (305, 306, 307) on the first screen 311 may be displayed as deactivated. The electronic device 100 may determine whether these areas (305, 306, 307) are activated based on the user's training level. For example, in the second screen 312, these areas (305, 306, 307) may become activated depending on the training level. Based on detecting a selection 305a of the phoneme chart status information 305, a third screen 313 may be displayed. The third screen 313 may be configured, for instance, based on the user's performance results in auditory training content for at least one phoneme. As shown in FIG. 3a, consonants that can form phonemes may be arranged vertically, and vowels may be arranged horizontally, but this arrangement is not limiting. Referring to FIG. 3a, syllables learned by the user (e.g., combinations of consonants and vowels) may be displayed at the intersection of the respective consonant and vowel. For a specific syllable, training content performed by the user may result in the syllable being displayed at the consonant/vowel intersection. Conversely, if training content for a specific syllable has not been performed, the intersection may remain blank. While the display of syllables at intersections is an example, objects indicating completed content for specific syllables, objects showing satisfactory content performance, or objects showing unsatisfactory performance may also be displayed. If the selection of a syllable displayed on the phoneme chart is detected, the corresponding speech sound for the selected syllable may be provided.

If the content performance result is satisfactory, the corresponding syllable may be displayed with a first attribute (here, the attribute could include color, transparency, saturation, brightness, etc., but is not limited to these). If the content performance result is unsatisfactory, the syllable may be displayed with an attribute different from the first attribute. For instance, in the third screen 313 of FIG. 3a, if the user, during phoneme training, mishears the syllable " 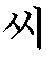 " " as " 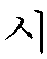 " and selects the answer corresponding to " 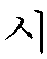 ," the electronic device 100 may display the syllable " 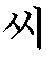 " at the intersection of the consonant " 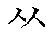 " and the vowel " 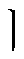 " in a color different from that of other syllables (e.g., red instead of black). In another example, the electronic device 100 may display the incorrect syllable " 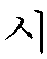 " in the cell where the consonant " 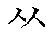 " and the vowel " 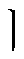 " meet, enabling the user to recognize how the syllable was misunderstood. In yet another example, only some of the phonemes that constitute the incorrect syllable (e.g., the misheard consonant or vowel) may be displayed at the intersection of the consonant " 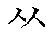 " and the vowel " 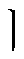 ." For instance, only the consonant " 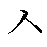 " may be displayed at the intersection of " 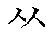 " and " 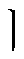 ." In this case, the consonant " 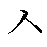 " may be displayed with the same or a different attribute as the correct syllables.

For instance, the third screen 313 may include a back navigation object 310a that allows returning to a previous state (e.g., the second screen 312). For example, the third screen 313 may include a consonant selection object 310b for selecting one or all consonants and/or a vowel selection object 310c for selecting one or all vowels. When a consonant (e.g., " 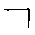 ") and a vowel (e.g., "T") are selected through the consonant selection object 310b and/or the vowel selection object 310c, a syllable (e.g., " 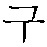 ") 310d combining the selected consonant and vowel, along with a playback object 310e, may be provided below the consonant selection object 310b and the vowel selection object 310c.

Referring again to the first screen 311 and/or the second screen 312, for instance, a quick access object 306 for sound illustration appreciation training, a quick access object 307 for sound drawing training, phoneme learning training information 308, and/or a quick access object 309 for training may be provided. Based on the designation of objects 306 or 307, the electronic device 100 may provide content for the corresponding training, with detailed descriptions to be provided later. The phoneme learning training information 308 may include information on the phoneme (e.g., a vowel or consonant such as " 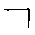 ") scheduled for current training (e.g., today) and the section to be performed (e.g., first half, second half, or comprehensive training).

Based on the selection of the training quick access object 309, a fourth screen 380, as shown in Figure 3b, may be provided. The fourth screen 380 may include objects 381, 382, 383, 384, 385, and 386 associated with multiple training contents. Based on the selection of at least one of the objects 381, 382, 383, 384, 385, and 386, the training content corresponding to the selected object may be provided. Alternatively, the objects 381, 382, 383, 384, 385, and 386 may be implemented as non-selectable. For example, the electronic device 100 may be configured to provide training content in a specified order. In such a case, the objects corresponding to content that cannot yet be performed, such as objects 382, 383, 384, and 385, may be displayed as deactivated, although this is exemplary and not limiting. In this scenario, based on the designation of an object 386 corresponding to "Proceed to Next Training" included in the fourth screen 380, the electronic device 100 may provide the training content identified based on the specified order. For example, the fourth screen 380 may also include an object 387 for transitioning the state to an initial screen, such as the first screen 311 or the second screen 312.

Figure 3c illustrates an example of a screen provided by the electronic device 100 according to an embodiment.

According to one embodiment, the electronic device 100 may provide a screen 390, as shown in Figure 3c. The screen 390 may be configured, for instance, based on the performance results of the user's auditory training content for at least one English phoneme. For example, consonants that can form English phonemes may be arranged vertically, and vowels may be arranged horizontally, although this is not limiting. There are no restrictions on the consonants and/or vowels (or combinations thereof) used to form English phonemes.

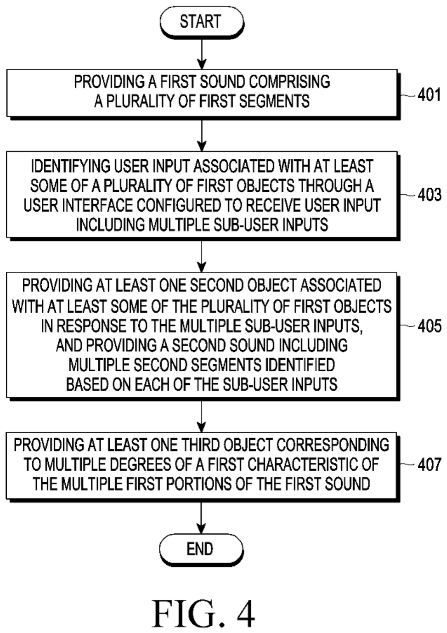

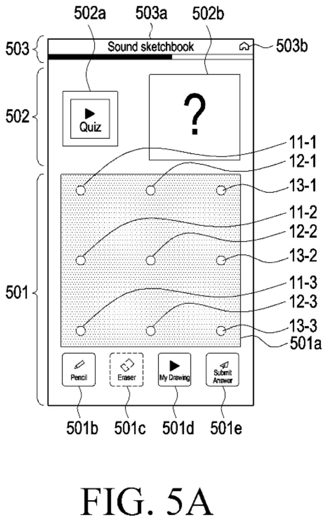

Figure 4 illustrates a method for providing content for auditory training according to an embodiment. The embodiment of Figure 4 will be described with reference to Figures 5a through 5i. Figures 5a through 5i illustrate content provided by an electronic device according to various embodiments.

Referring to Figure 4, the electronic device 100 may, in operation 401, provide a first sound (or referred to as a test sound) comprising a plurality of first segments. Here, the provision of the first sound may involve, for example, outputting sound through a speaker included in the electronic device 100 or transmitting data to trigger sound output via an external speaker connected to the electronic device 100 either by wired or wireless means, as understood by those skilled in the art. For instance, the electronic device 100 may provide (e.g., display) a user interface, as shown in Figure 5a, and may provide the first sound in conjunction with the provision of the user interface. It should be understood by those skilled in the art that there are no restrictions on the timing for initiating the provision of the user interface.

Referring to Figure 5a, the user interface may include multiple regions: first region 501, second region 502, and third region 503, although this is merely exemplary. The third region 503 may include a training title 503a and a home object 503b for returning to the initial screen 311 or 312. The second region 502 may include a first play object 502a for requesting the provision (e.g., playback) of the first sound (or referred to as a test sound) for drawing training and a pattern display area 502b for displaying objects corresponding to the first sound. The first region 501 may include a visual pattern input area 501a, a drawing object (e.g., pencil) 501b, an erasing object (e.g., eraser) 501c, a second play object (e.g., "My Drawing") 501d, and a correct answer submission object (e.g., "Submit Answer") 501e. The visual pattern input area 501a may include multiple first objects 11-1, 11-2, 11-3, 12-1, 12-2, 12-3, 13-1, 13-2, 13-3 for selecting one of the multiple designated values related to the characteristics of the test sound in its multiple segments. For example, each of the multiple first objects 11-1, 11-2, 11-3, 12-1, 12-2, 12-3, 13-1, 13-2, 13-3 may correspond to one of the designated values associated with the characteristics of the sound. The multiple first objects may be arranged in a grid (e.g., 3 rows × 3 columns). In this case, the first objects in each column may correspond to different values, and the first objects in each row may correspond to the same value. For instance, the first objects in the first row 11-1, 12-1, 13-1 may correspond to a first value (e.g., a frequency of the first magnitude or a first timbre). The first objects in the second row 11-2, 12-2, 13-2 may correspond to a second value (e.g., a frequency of the second magnitude or a second timbre). The first objects in the third row 11-3, 12-3, 13-3 may correspond to a third value (e.g., a frequency of the third magnitude or a third timbre).

The first play object 502a may be configured to trigger the provision of the test sound upon selection. On the correct visual pattern display area 502b, an object corresponding to the correct visual pattern associated with the properties of the test sound may be displayed in response to the selection of the correct answer submission object 501e (or a request for the correct visual pattern). Meanwhile, before the selection of the correct answer submission object 501e (or the request for the correct visual pattern) is confirmed, the correct visual pattern display area 502b may, for instance, display a preview prevention object (e.g., a question mark) as shown in Figure 5a. It should be noted that the provision of the correct visual pattern display area 502b as a region distinct from the visual pattern input area 501a, as illustrated in Figure 5a, is merely exemplary. In other implementations, the user interface may be designed without including the correct visual pattern display area 502b and instead incorporate only the visual pattern input area 501a. In such cases, the object with the correct visual pattern may be displayed on the visual pattern input area 501a either alongside the object with the visual pattern input by the user or sequentially in relation to the object with the user-input visual pattern. The implementation method is not limited to these examples.

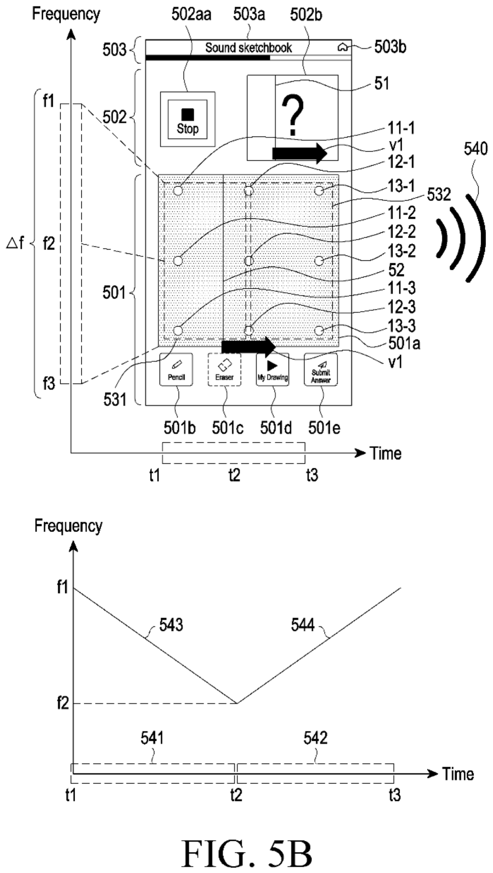

Upon confirming the selection of the first play object 502a, the electronic device 100 may provide the test sound 540. Based on the initiation of the provision of the test sound 540, a pause object 502aa may replace the first play object 502a on display; however, this is not limiting. The test sound 540 may have at least one characteristic (e.g., frequency, timbre, overtone density, and/or volume, though not limited thereto). At least some of the characteristics of the test sound 540 may be designed for auditory training. For instance, in the example of Figure 5b, the frequency of the first portion 543 of the test sound 540 may change from f1 to f2 during the first time interval 541 between the first time point t1 and the second time point t2 (e.g., linearly, though the mode of variation is not limited). Similarly, the frequency of the second portion 544 of the test sound 540 may change from f2 to f1 during the second time interval 542 between the second time point t2 and the third time point t3. It should be noted that frequency is merely an exemplary characteristic, and the type of characteristic is not limited. Furthermore, while Figure 5b illustrates a characteristic (frequency) undergoing change, it is understood by those skilled in the art that a characteristic may remain constant over certain time intervals.

As the test sound 540 is provided, a first indicator 51, visually representing the playback position of the test sound 540, may be depicted as moving across the pattern display area 502b at, for instance, a speed v1. The speed v1 may be set based on the test difficulty level, which will be described in detail later. For example, the horizontal length of the pattern display area 502b may correspond to the entire duration of the time intervals 541 and 542 during which the test sound 540 is provided. At the initiation of the provision of the test sound 540, the first indicator 51 may be depicted as starting to move from the left edge of the pattern display area 502b. As time progresses and the test sound 540 continues to play, the first indicator 51 may also be depicted as moving proportionally to the cumulative time of the test sound 540 being provided, progressing toward the right. At the third time point t3, the first indicator 51 may be depicted as reaching the right edge of the pattern display area 502b.

For example, a second indicator 52 may be depicted as moving at the same speed v1 as the first indicator 51 during the provision of the test sound 540. The horizontal length of the visual pattern input region 501a may correspond to the total duration of the time intervals 541 and 542 during which the test sound 540 is provided. For instance, the first section 531 of the visual pattern input region 501a may correspond to the time interval 541, while the second section 532 may correspond to the time interval 542. At the initiation of the test sound 540, the second indicator 52 may be depicted as starting to move from the left edge of the visual pattern input region 501a. As time progresses and the test sound 540 continues to play, the second indicator 52 may also be depicted as moving proportionally to the cumulative time of the test sound 540 being provided, progressing toward the right. At the third time point t3, the second indicator 52 may be depicted as reaching the right edge of the visual pattern input region 501a. Thus, the user can recognize which part of the visual pattern input region 501a corresponds to the auditory test sound 540 and where to input a visual pattern object matching the sound. Meanwhile, the provision of the indicators 51 and 52 is merely exemplary, and it is understood that the representation of at least some of the indicators 51 and 52 may be omitted or skipped depending on the difficulty level of the auditory training.

The vertical direction of the visual pattern input region 501a may, for instance, correspond to characteristics of the sound. In the example illustrated in Figure 5b, the vertical direction of the visual pattern input region 501a may correspond to the frequency range f1 to f3. For example, the top section of the visual pattern input region 501a (or objects 11-1, 12-1, 13-1 positioned at the highest level) may correspond to the frequency f1. The middle section (or objects 11-2, 12-2, 13-2 positioned at the center) may correspond to the frequency f2, and the bottom section (or objects 11-3, 12-3, 13-3 positioned at the lowest level) may correspond to the frequency f3. The frequency boundaries (f1, f3) and/or the frequency range Δf may be predefined or set based on the training difficulty level; however, these settings are not limited, and a detailed explanation will be provided later. As shown in Figure 5b, while listening to the test sound 540, the user can prepare to input an object with a visual pattern corresponding to the test sound 540 into the visual pattern input region 501a.

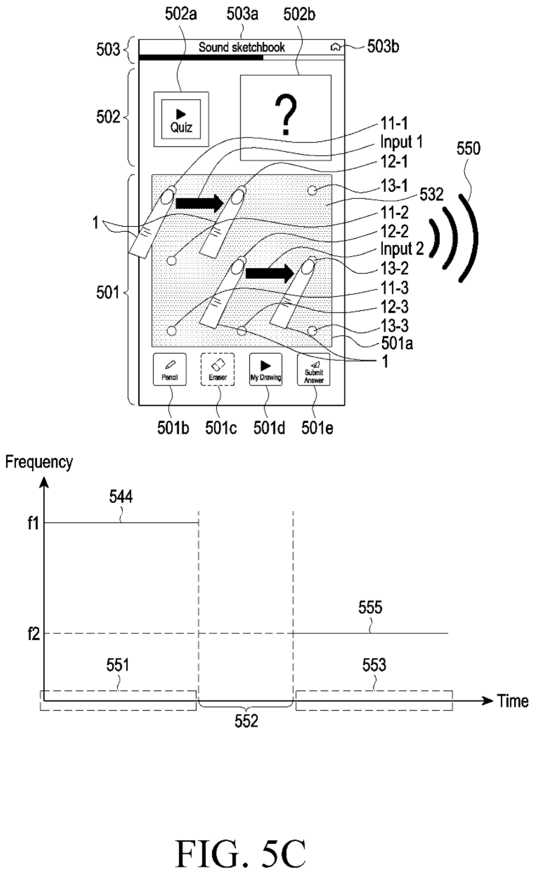

Referring again to Figure 4, in operation 403, the electronic device 100 can identify user input associated with at least some of a plurality of first objects (e.g., objects 11-1, 11-2, 11-3, 12-1, 12-2, 12-3, 13-1, 13-2, 13-3) through a user interface configured to receive user input including multiple sub-user inputs. In operation 405, the electronic device 100 can provide at least one second object (e.g., an object with a visual pattern) associated with at least some of the plurality of first objects (e.g., objects 11-1, 11-2, 11-3, 12-1, 12-2, 12-3, 13-1, 13-2, 13-3) in response to the multiple sub-user inputs. Additionally, the electronic device can provide a second sound including multiple second segments identified based on each of the sub-user inputs. For example, as shown in Figure 5c, the user can use a finger 1 to input a visual pattern corresponding to the test sound 540 on the visual pattern input region 501a. The user may input the visual pattern while an object 501b, intended for visual pattern input, is activated. However, this is not limiting. While the object 501b for visual pattern input is activated, the object 501c for deleting an already-input visual pattern may be deactivated, but this is merely exemplary. For instance, as depicted in Figure 5c, during the first time interval 551, the user may input a first user input (input1) (e.g., a drag, but not limited to this) from object 11-1 to object 12-1. During the second time interval 552, the user may release the touch on object 12-1 with finger 1, move finger 1, and touch object 12-2. During the third time interval 553, the user may input a second user input (input2) from object 12-2 to object 13-2.

The electronic device 100 can provide a sound 550 based on at least one user input. For example, a first segment 554 of sound 550 may have characteristics corresponding to the first user input (input1). The first user input (input1) may, for instance, be a rightward movement from object 11-1 to object 12-1. Accordingly, the first segment 554 provided during the first time interval 551 may consist of a sub-segment corresponding to object 11-1 with a first frequency (f1), sub-segments corresponding to multiple points between objects 11-1 and 12-1, each having the first frequency (f1), and a sub-segment corresponding to object 12-1 with the first frequency (f1). Subsequently, during the second time interval 552, in response to the absence of user input, the electronic device 100 may refrain from outputting sound. For instance, a second segment 555 of sound 550 may have characteristics corresponding to the second user input (input2). The second user input (input2) may, for example, be a rightward movement from object 12-2 to object 13-2. Accordingly, the second segment 555 provided during the third time interval 553 may consist of a sub-segment corresponding to object 12-2 with a second frequency (f2), sub-segments corresponding to multiple points between objects 12-2 and 13-2, each having the second frequency (f2), and a sub-segment corresponding to object 13-2 with the second frequency (f2). As described above, the user can input a visual pattern corresponding to the test sound 540 while listening to the sound 550 that corresponds to the points (or transitions between points) they are entering.

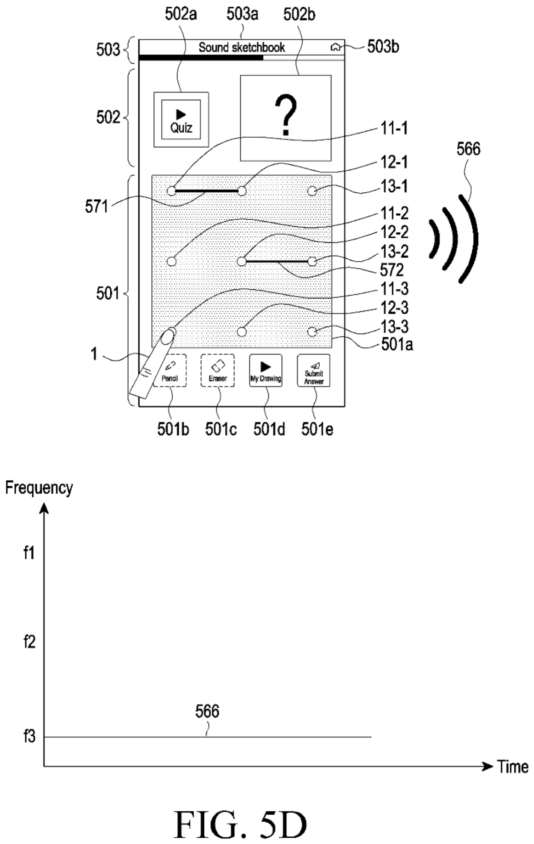

Referring to Figure 5d, objects 571 and 572 with visual patterns identified based on user inputs (input1 and input2) as shown in Figure 5c may be displayed. Meanwhile, even after the objects 571 and 572 are displayed, the user can continue to provide additional inputs. For instance, as shown in Figure 5d, if the user touches and holds object 11-3, the electronic device 100 may provide a sound 566 corresponding to object 11-3. The sound 566 may have a third frequency (f3) corresponding to object 11-3. On the other hand, if no event for displaying an object with a visual pattern is detected, the device may be configured such that no object, like the visual patterns 571 and 572, is displayed even when a user input is identified. For example, the event for displaying an object with a visual pattern could involve identifying a user input that designates at least two of the objects (11-1, 11-2, 11-3, 12-1, 12-2, 12-3, 13-1, 13-2, 13-3) as a start point and an endpoint. However, this is merely exemplary, and there are no limitations on the types of events for displaying objects with visual patterns. For instance, the electronic device 100 may temporarily display an object corresponding to the user's touch point and cease displaying the object if no event is detected, however, this is not restrictive.

Meanwhile, the provision of objects 571 and 572 with visual patterns corresponding to user inputs (input1, input2) and the associated sounds 554 and 555 is merely exemplary. Depending on the implementation, the electronic device 100 may provide objects 571 and 572 with visual patterns corresponding to user inputs (input1, input2) without providing the sounds 554 and 555 associated with the objects 571 and 572. This would be understood by those skilled in the art.

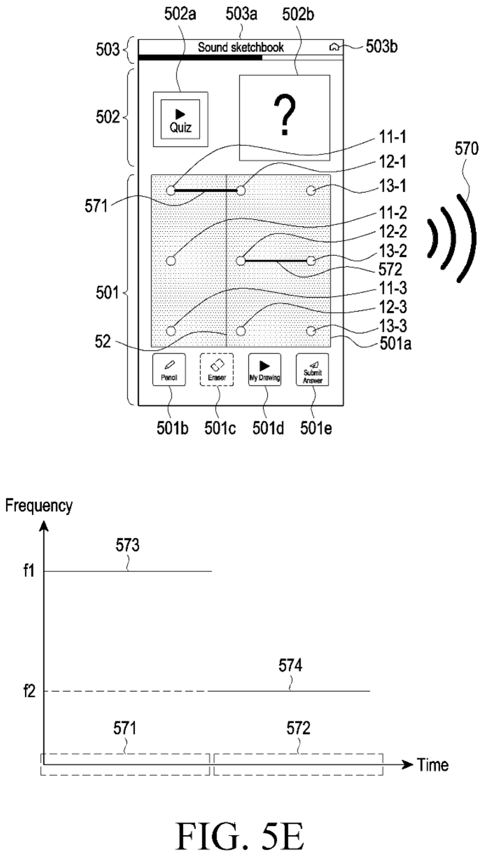

The user may, for example, select the second playback object 501d if they wish to listen to the sound corresponding to the object they created. As shown in Figure 5e, based on the selection of the second playback object 501d, a stop playback object 501dd may replace the second playback object 501d, though this is not limiting. The electronic device 100, based on the selection of the second playback object 501d, may provide a sound 570 corresponding to the objects 571 and 572 with visual patterns. For instance, the first part 573 of the sound 570 may be provided during the first time interval 571. The first part 573 of the sound 570 may correspond to the first part 571 of the object. Since the first part 571 of the object corresponds to the first frequency f1, the first part 573 of the sound 570 may have the first frequency f1. Similarly, the second part 574 of the sound 570 may be provided during the second time interval 572. The second part 574 of the sound 570 may correspond to the second part 572 of the object with visual patterns. Since the second part 572 of the object corresponds to the second frequency f2, the second part 574 of the sound 570 may have the second frequency f2. The duration of the first time interval 571 may be, for example, substantially the same as the duration of the first time interval 541 in which the first part 543 of the test sound 540 in Figure 5b is provided, though this is not limiting. Similarly, the duration of the second time interval 572 may be substantially the same as the duration of the second time interval 542 in which the second part 544 of the test sound 540 in Figure 5b is provided, though this is not limiting. As a result, the user can confirm whether the visual pattern they created corresponds to the test sound 540. If the user recognizes that the visual pattern they created does not correspond to the test sound 540, they may activate (e.g., touch) the erase object 501c to delete at least part of the objects 571 and 572 with visual patterns being displayed and input a new object with a different visual pattern.

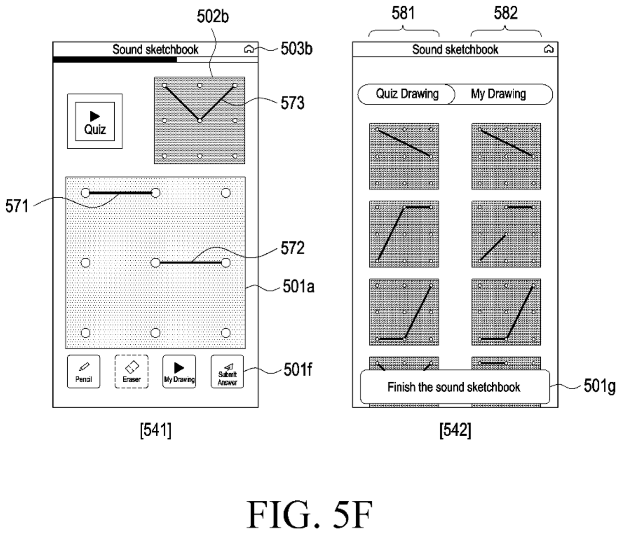

Referring again to Figure 4, the electronic device 100 may, in operation 407, provide at least one third object (e.g., an object with a correct visual pattern) corresponding to multiple degrees of a first characteristic of the multiple first portions of the first sound. For example, the electronic device 100, based on the selection of the correct confirmation object 501e, may display an object 573 with a correct visual pattern corresponding to the test sound 540, as shown in Figure 5f. Based on the selection of the correct confirmation object 501e, an object 501f for progressing to the next test may replace the correct confirmation object 501e, though this is not limiting. Meanwhile, the expression of the object 573 with the correct visual pattern may be performed based on various events (e.g., the passage of a specified time, or a match between an object with a visual pattern based on user input and the correct answer), in addition to the designation of the object 501e, without limitation. Additionally, as described above, in another implementation, the electronic device 100 may display both the object 571, 572 with a visual pattern identified based on user input and the object with the correct visual pattern in the visual pattern input area 501a. Referring to the right side of Figure 5f, objects 581 with correct visual patterns corresponding to the test sounds of previously performed auditory training by the user and objects 582 with visual patterns identified by user input may be displayed together. Furthermore, based on the selection of an object 501g associated with the termination of auditory training based on auditory and visual patterns, the electronic device 100 may finish the training.

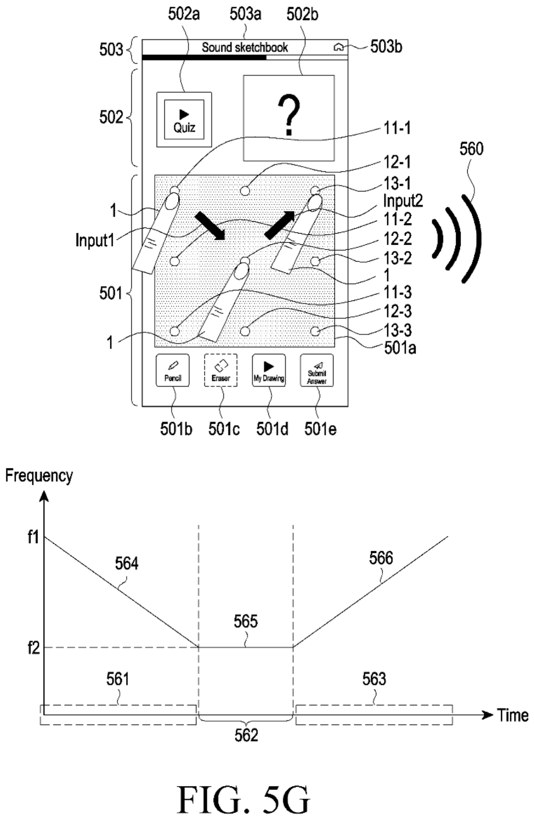

In Figure 5g, user input corresponding to the correct object for the test sound 540 may be identified. For instance, as shown in Figure 5g, the user can input a visual pattern corresponding to the test sound 540 into the visual pattern input area 501a using a finger 1. For example, as illustrated in Figure 5g, during the first time interval 561, the user can input the first user input (input1), such as a drag action (though not limited to this), from object 11-1 to object 12-2. During the second time interval 562, the user may maintain the touch of finger (1) on object 12-1. During the third time interval 563, the user can input the second user input (input2) from object 12-2 to object 13-1.

The electronic device 100 can provide a sound 560 based on at least one user input. For example, the first portion 564 of sound 560 may have characteristics corresponding to the first user input (input1). The first user input (input1) could involve a downward-right movement from object 11-1 to object 12-2. Accordingly, the first portion 564, provided during the first time interval 561, may include a sub-portion corresponding to object 11-1 with the first frequency (f1), sub-portions with multiple intermediate frequencies (frequencies between f1 and f2) corresponding to several points between objects 11-1 and 12-2, and a sub-portion corresponding to object 12-2 with the second frequency (f2). Subsequently, during the second time interval 552, based on the user input being maintained on object 12-2, the electronic device 100 may provide the second portion 565 of sound 560, which corresponds to the second frequency (f2) of object 12-2. For example, the third portion 566 of sound 560 may have characteristics corresponding to the second user input (input2). The second user input (input2) may involve an upward-right movement from object 12-2 to object 13-1. Accordingly, the third portion 566, provided during the third time interval 563, may include a sub-portion corresponding to object 12-2 with the second frequency (f2), sub-portions with multiple intermediate frequencies (frequencies between f2 and f1) corresponding to several points between objects 12-2 and 13-1, and a sub-portion corresponding to object 13-1 with the first frequency (f1). As described above, the user can listen to sound 560 corresponding to the points (or transitions between points) they are inputting and use it as a reference to input objects with visual patterns corresponding to the test sound 540.

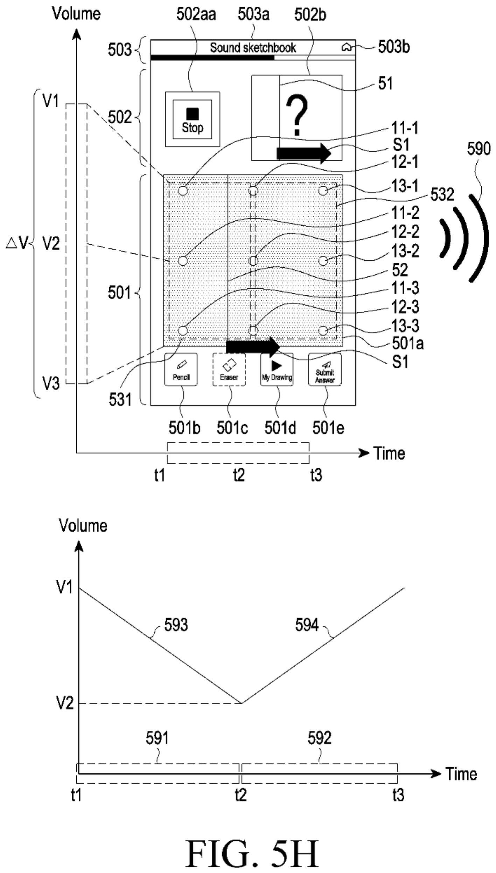

Figure 5h illustrates a diagram explaining a sound test based on characteristics other than frequency, according to an embodiment.

For example, in the examples of Figures 5b through 5e, frequency as one of the characteristics of the test sound can be set as the target feature for training. In contrast, in the embodiment shown in Figure 5h, volume, as one of the characteristics of the test sound, can be set as the target feature for training. In Figure 5h, the vertical direction of the visual pattern input area 501a can correspond to a range of volumes, such as V1 to V3. For instance, the top of the visual pattern input area 501a (or objects positioned at the topmost part, e.g., 11-1, 12-1, 13-1) may correspond to the volume level V1. The center of the visual pattern input area 501a (or objects positioned in the center, e.g., 11-2, 12-2, 13-2) may correspond to the volume level V2, and the bottom of the visual pattern input area 501a (or objects positioned at the lowest part, e.g., 11-3, 12-3, 13-3) may correspond to the volume level V3. Volume thresholds V1, V3 and/or volume ranges ΔV can be predetermined or adjusted based on training difficulty, though they are not limited in their implementation. Detailed explanations regarding this configuration will be provided later. As illustrated in Figure 5h, the user can prepare to input objects with visual patterns corresponding to the test sound 593, 594 on the visual pattern input area 501a while listening to the respective portions 593, 594 of the test sound provided during time intervals 591, 592. It should also be understood by those skilled in the art that, in addition to characteristics such as frequency or volume, which can be expressed as values, all sound characteristics can be utilized without limitation for auditory training.

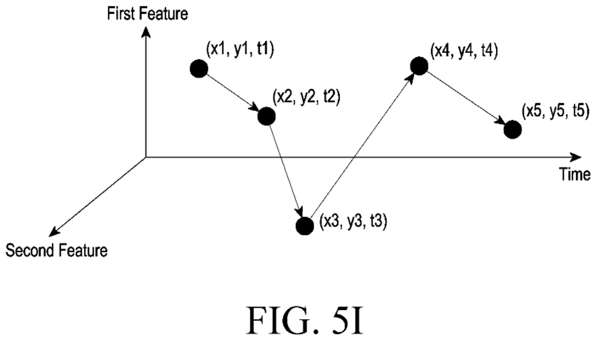

Figure 5i illustrates a diagram explaining auditory training based on multi-dimensional characteristics according to an embodiment.

For example, multiple characteristics of the test sound (e.g., a first characteristic and a second characteristic) can be designed to be used in auditory training. The test sound, at a first point in time t1, may include a portion having a first characteristic at a degree of x1 and a second characteristic at a degree of y1. The test sound, between the first point in time t1 and a second point in time t2, may include a portion having a first characteristic changing from x1 to x2 and a second characteristic changing from y1 to y2. The test sound, between the second point in time t2 and a third point in time t3, may include a portion having a first characteristic changing from x2 to x3 and a second characteristic changing from y2 to y3. The test sound, between the third point in time t3 and a fourth point in time t4, may include a portion having a first characteristic changing from x3 to x4 and a second characteristic changing from y3 to y4. The test sound, between the fourth point in time t4 and a fifth point in time t5, may include a portion having a first characteristic changing from x4 to x5 and a second characteristic changing from y4 to y5.

The electronic device 100 may provide a user interface for inputting a visual pattern corresponding to the test sound. For example, the user interface may include a first sub-user interface for inputting a projective visual pattern part corresponding to a first characteristic and a second sub-user interface for inputting a projective visual pattern part corresponding to a second characteristic. For instance, the user may input the projective visual pattern part for the first characteristic through the first sub-user interface and input the projective visual pattern part for the second characteristic through the second sub-user interface. In another implementation, the user interface may be configured to allow the input of three-dimensional information (first characteristic, second characteristic, and time). For example, when the electronic device 100 provides a VR, AR, or MR environment, the electronic device 100 may recognize the position of an input device (or a part of the user's body) in three-dimensional space. Accordingly, based on the trajectory of the input device (or part of the user's body) in three-dimensional space, the electronic device 100 may recognize a visual pattern defined in three dimensions. Meanwhile, it should be understood by those skilled in the art that two-dimensional visual patterns described above may also be recognized based on the VR, AR, or MR environment.

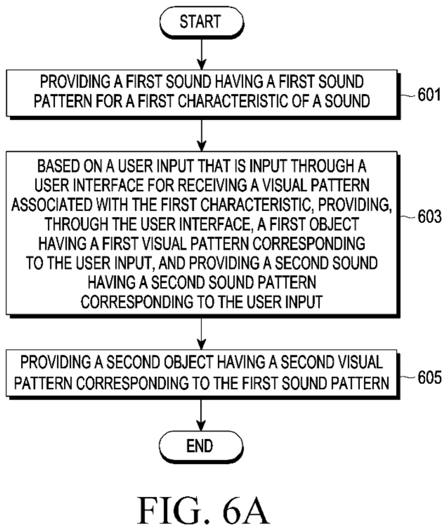

Figure 6a illustrates a method for providing auditory training content according to one embodiment.

In this embodiment, the electronic device 100, in operation 601, may provide a first sound having a first auditory pattern corresponding to a first characteristic of sound. For example, if the first characteristic is frequency, the first auditory pattern may be a sound in which the frequency changes over time; however, this is merely exemplary, and there are no limitations on the types or numbers of characteristics used to form the pattern. The first sound here may serve as a test sound for evaluating the user's hearing ability (and/or for improving auditory capability). For instance, the electronic device 100 may provide, as a test sound, the first sound 540 with the first auditory pattern (e.g., a pattern where the frequency decreases from f1 to f2 during the first period 541 and increases from f2 to f1 during the second period 542) described in Figure 5b.

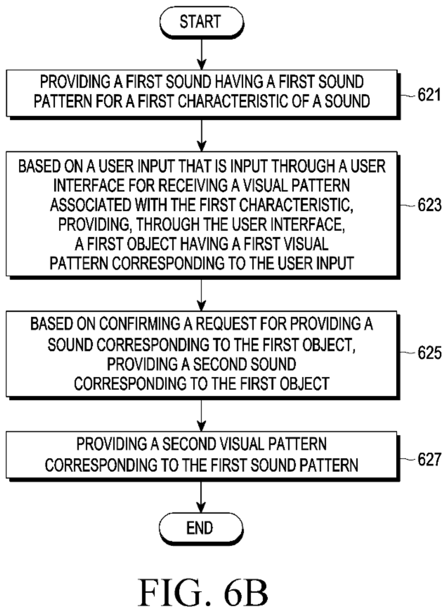

The electronic device 100, in operation 603, may provide a first object having a first visual pattern corresponding to user input (e.g., the first user input input1 and the second user input input2 in Figure 5c) through a user interface for inputting a visual pattern associated with the first characteristic (e.g., the visual pattern input area 501a in Figure 5c). Additionally, the electronic device may provide a second sound having a second auditory pattern corresponding to the user input (e.g., the sound 554, 555 in Figure 5c). For example, the timing and/or duration of the second sound (e.g., the sound 554, 555 in Figure 5c) may be substantially synchronized with the detection timing and/or duration of the first user input input1 and the second user input input2; however, this synchronization is not mandatory. Alternatively or additionally, instead of or in addition to the second sound (e.g., the sound 554, 555 in Figure 5c) synchronized with the first and second user inputs, the electronic device may provide a sound corresponding to the first object having the first visual pattern (e.g., the sound 570 in Figure 5e). This will be described with reference to Figure 6b. It should also be noted that the provision of the second sound having the second auditory pattern corresponding to the user input (e.g., the sound 554, 555 in Figure 5c) may be omitted. In operation 605, the electronic device 100 may provide a second object having a second visual pattern corresponding to the first auditory pattern (e.g., the object 573 in Figure 5f, also referred to as a correct visual pattern object). For instance, based on the selection of the correct confirmation object (e.g., "Submit Answer" object 501e), the device may provide the second object having the second visual pattern (e.g., the object 573 in Figure 5f), though the trigger for providing this second object is not limited to this example.

Figure 6b illustrates a method for providing auditory training content according to an embodiment.

According to the embodiment, in operation 621, the electronic device 100 may provide a first sound having a first auditory pattern for a first characteristic of the sound. In operation 623, the electronic device 100, through a user interface for inputting a visual pattern associated with the first characteristic (e.g., the visual pattern input area 501a in Figure 5c), may provide a first object having a first visual pattern corresponding to user input (e.g., the first user input input1 and the second user input input2 in Figure 5c) through the user interface. For example, in operation 625, the electronic device 100 may provide a second sound corresponding to the first object (e.g., the objects 571, 572 in Figure 5d) based on detecting a request to play the sound corresponding to the first object. For instance, based on the selection of the second playback object (e.g., "My Drawing" object 501d), the electronic device 100 may provide the second sound (e.g., the sound 570 in Figure 5e) corresponding to the first object (e.g., the objects 571, 572 in Figure 5d). However, the trigger for providing this sound is not limited to this example. In operation 627, the electronic device 100 may provide a second object having a second visual pattern corresponding to the first auditory pattern (e.g., the object 573 in Figure 5f, also referred to as a correct visual pattern object). For instance, based on the selection of the correct confirmation object (e.g., "Submit Answer" object 501e), the device may provide the second object having the second visual pattern (e.g., the object 573 in Figure 5f), though the trigger for providing this second object is not limited to this example.

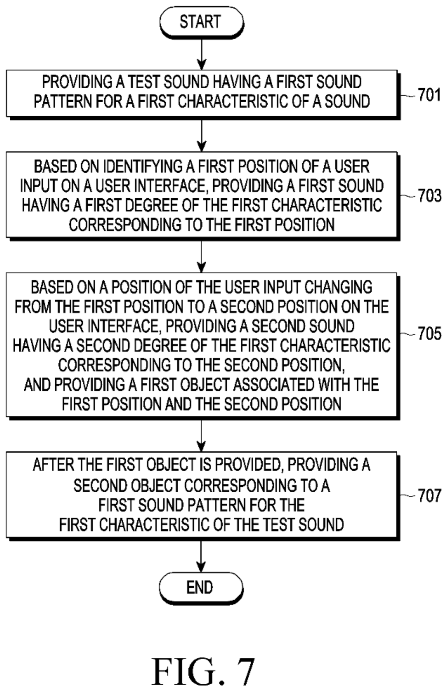

Figure 7 illustrates a method for providing auditory training content according to an embodiment.

According to the embodiment, in operation 701, the electronic device 100 may provide a test sound having a first auditory pattern for a first characteristic of the sound. In operation 703, the electronic device 100 may provide a first sound having a first degree of the first characteristic corresponding to a first position on the user interface, based on identifying the user input at the first position. For example, as described with reference to Figure 5b, the vertical direction of the visual pattern input area 501a may correspond to a characteristic of the sound. In the example of Figure 5b, the vertical direction of the visual pattern input area 501a corresponds to the frequency range from f1 to f3. Accordingly, based on user input at a first point in the visual pattern input area 501a, a sound with the characteristic value (e.g., frequency) corresponding to the first point may be provided. This sound may, for instance, be provided while the user input is maintained at the first point, though this is not limiting. For instance, as described with reference to Figure 5g, the user may input a first user input (input1), such as a drag operation, from object 11-1 to object 12-2 during the first time interval (561). Based on the user input at object 11-1, a sound with the frequency f1 corresponding to object 11-1 may be provided. In operation 705, the electronic device 100 may provide a second sound having a second degree of the first characteristic corresponding to a second position on the user interface, based on the user input moving from the first position to the second position. Simultaneously, the device may provide a first object associated with the first position and the second position. For example, as shown in Figure 5g, the first user input (input1), such as a drag operation, moves the user input from object 11-1 to object 12-2. Accordingly, the electronic device 100 may provide a sound with the frequency f2 corresponding to object 12-2. The device may also provide at least one intermediate sound with a characteristic corresponding to a position between the first position (object 11-1) and the second position (object 12-2), during the time interval between the provision of the sound corresponding to the first position and the sound corresponding to the second position. For example, as described in Figure 5g, the sound 564 may include the sound corresponding to the frequency f1 at the first position, the sound corresponding to the frequency f2 at the second position, and intermediate sounds between the two sub-sounds. The intermediate sounds may, for example, have frequencies between f1 and f2. While the frequencies of the intermediate sounds are shown as changing linearly, this is merely illustrative and not limiting. In operation 707, after the first object is provided, the electronic device 100 may provide a second object corresponding to the first auditory pattern for the first characteristic of the test sound-for example, a correct object.

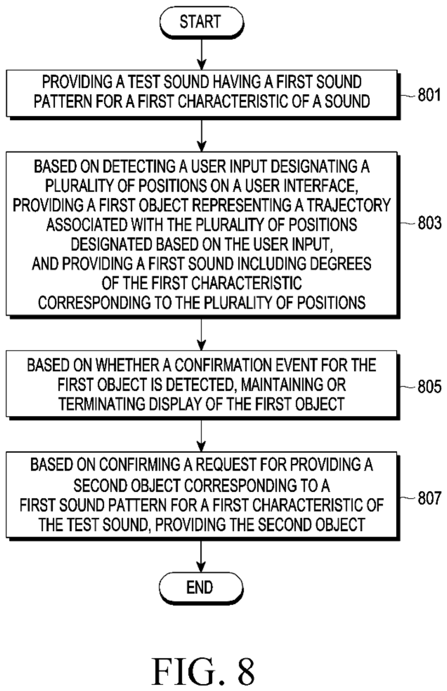

Figure 8 illustrates a method for providing auditory training content according to an embodiment.

According to the embodiment, in operation 801, the electronic device 100 may provide a test sound having a first auditory pattern for a first characteristic of the sound. In operation 803, the electronic device 100 may detect user input designating multiple positions on the user interface. Based on the designated multiple positions, the device may provide a first object representing a trajectory associated with the user input while providing a first sound that includes parts corresponding to the degrees of the first characteristic at the multiple positions. In operation 805, the electronic device 100 may determine whether to maintain or discontinue the display of the first object based on whether a confirmation event for the first object is detected. For example, if the trajectory associated with the user input (e.g., the first object) corresponds to multiple reference points on the user interface (e.g., the multiple first objects 11-1, 11-2, 11-3, 12-1, 12-2, 12-3, 13-1, 13-2, 13-3 in Figure 5a) through contact, passage, or proximity, the device may confirm that the first object's confirmation event has occurred. Upon detecting the confirmation event, the electronic device 100 may maintain the display of the whole or part of the first object. For instance, the device may maintain the display of at least one trajectory connecting the multiple reference points on the user interface (e.g., as described in Figure 5a). However, there are no restrictions on this behavior. If no confirmation event is detected for the first object, the electronic device 100 may discontinue the display of the trajectory. For example, if the trajectory does not correspond to multiple reference points, the device may cease displaying the trajectory based on detecting user input termination (e.g., releasing a touch), following the trajectory's display. In operation 807, the electronic device 100 may provide a second object corresponding to the first auditory pattern for the first characteristic of the test sound, based on confirming a request to provide the second object. For example, when a user determines that the input object corresponds to their intended visual pattern, they may request the provision of the second object corresponding to the first auditory pattern (e.g., by selecting the correct confirmation object such as Submit Answer 501e). Upon confirming the request (e.g., detecting the selection of Submit Answer 501e), the electronic device 100 may provide the second object corresponding to the first auditory pattern.

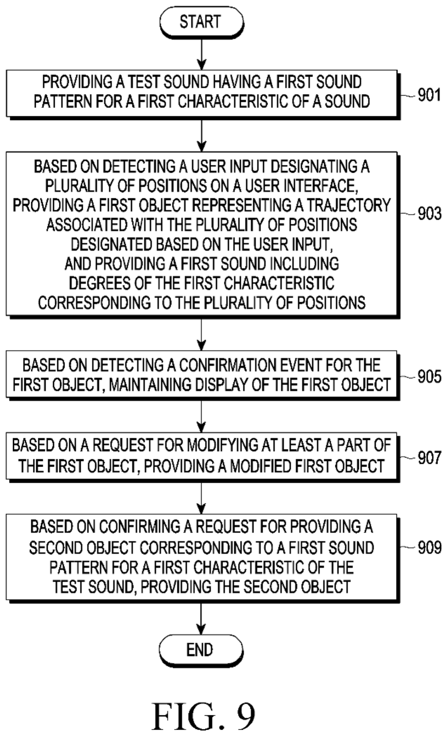

Figure 9 illustrates a method for providing auditory training content according to an embodiment.

According to the embodiment, in operation 901, the electronic device 100 may provide a test sound having a first auditory pattern for a first characteristic of the sound. In operation 903, the electronic device 100 may detect user input designating multiple positions on the user interface. Based on the designated positions, the device may provide a first object representing a trajectory associated with the user input while providing a first sound that includes parts corresponding to the degrees of the first characteristic at the designated positions. In operation 905, based on detecting a confirmation event for the first object, the electronic device 100 may maintain the display of the first object, either in whole or in part. In operation 907, based on a modification request for at least part of the first object, the electronic device 100 may provide a modified first object. For example, upon confirming the selection of a deletion object (e.g., eraser 501c), the electronic device 100 may change the mode of the user interface from object input mode to object deletion mode (or modification mode). In object input mode, objects corresponding to user input on the user interface (e.g., the visual pattern input area 501a) may be displayed. In object deletion mode, previously displayed objects on the user interface (e.g., the visual pattern input area 501a) may be deleted (or hidden) in response to user input. For example, the entire previously displayed object may be deleted based on user input. Alternatively, only part of the previously displayed object may be deleted based on user input. The unit of the deleted portion could correspond to the segments between reference points, but this is illustrative, and there are no limitations on the unit of deletion. If the selection of a drawing object (e.g., pencil 501b) is confirmed, the electronic device 100 may switch the user interface mode back from deletion mode (or modification mode) to object input mode. In operation 909, based on confirming a request to provide a second object corresponding to the first auditory pattern for the first characteristic of the test sound, the electronic device 100 may provide the second object. For example, upon confirming a user selection of a correct confirmation object (e.g., Submit Answer 501e), the electronic device 100 may provide the second object corresponding to the first auditory pattern.

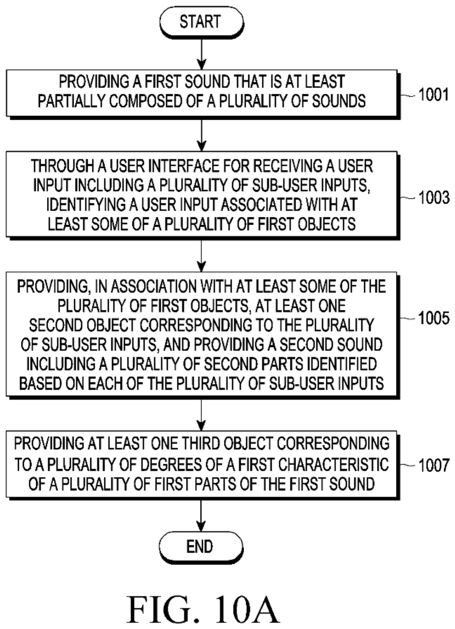

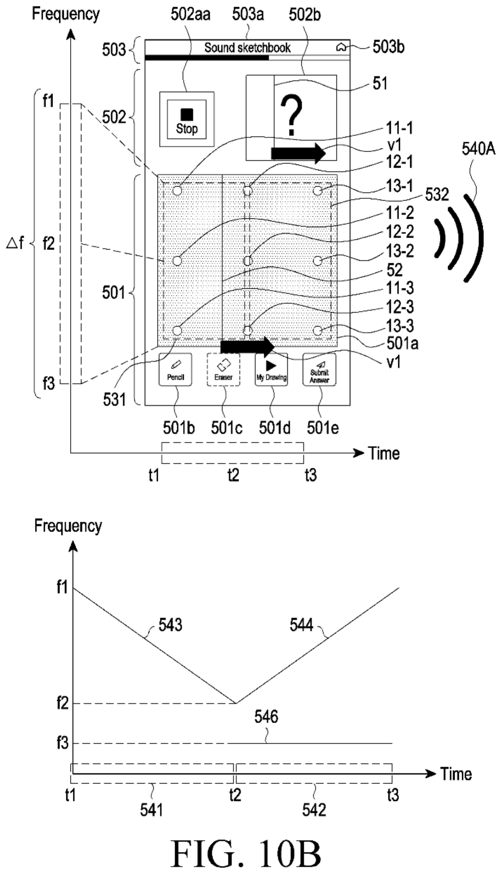

Figure 10a illustrates a method for providing auditory training content according to an embodiment, which will be explained with reference to Figure 10b. Figure 10b describes multiple sounds according to an embodiment.

In this embodiment, in operation 1001, the electronic device 100 may provide a first sound composed of multiple sounds at least in part. For example, as illustrated in Figure 10b, the frequency of the first portion 543 of the test sound 540A may change from f1 to f2 during the first time interval 541 between time points t1 and t2. The frequency of the second portion 544 of the test sound 540A may change from f2 to f1 during the second time interval 542 between time points t2 and t3. Additionally, the frequency of the third portion 546 of the test sound 540A may remain constant at f3 during the same time interval 542 between time points t2 and t3. As shown in Figure 10b, during the interval between t2 and t3, multiple sounds (e.g., the second portion 544 and the third portion 546) may be provided at least partially simultaneously. In operation 1003, the electronic device 100 may detect user input associated with at least some of the first objects through a user interface for receiving multiple sub-user inputs. In operation 1005, the electronic device 100 may provide at least one second object associated with at least some of the first objects and provide a second sound including multiple second portions corresponding to the multiple sub-user inputs. For example, while listening to the multiple sounds (e.g., the second portion 544 and the third portion 546) during the interval between t2 and t3, the user may be required to input multiple objects corresponding to the multiple sounds in the second portion 532 of the user interface corresponding to the interval between t2 and t3. These objects may include one connecting objects 12-2 and 12-1 and another connecting objects 12-3 and 13-3. In operation 1007, the electronic device 100 may provide at least one third object, such as a correct answer object, corresponding to the degrees of the first characteristic of the multiple first portions of the first sound.

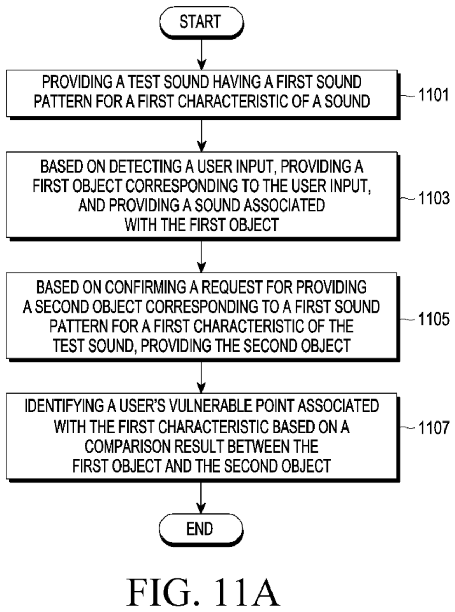

Figure 11a illustrates a method for providing auditory training content according to an embodiment.

In this embodiment, in operation 1101, the electronic device 100 may provide a test sound having a first auditory pattern corresponding to a first characteristic of sound. In operation 1103, the electronic device 100 may detect user input and, based on the detection, provide a first object corresponding to the user input while also providing a sound associated with the first object. In operation 1105, the electronic device 100 may provide a second object corresponding to the first auditory pattern of the first characteristic of the test sound, based on confirming a request for the provision of the second object. In operation 1107, the electronic device 100 may identify a user's vulnerable points related to the first characteristic based on a comparison between the first object and the second object. For example, the electronic device 100 may identify the user's vulnerable points related to the first characteristic based on mismatched portions between the first object and the second object. These vulnerable points may be represented as values (e.g., frequency values or ranges in the case of frequency) associated with the first characteristic; however, the method of representation is not limited to this example.

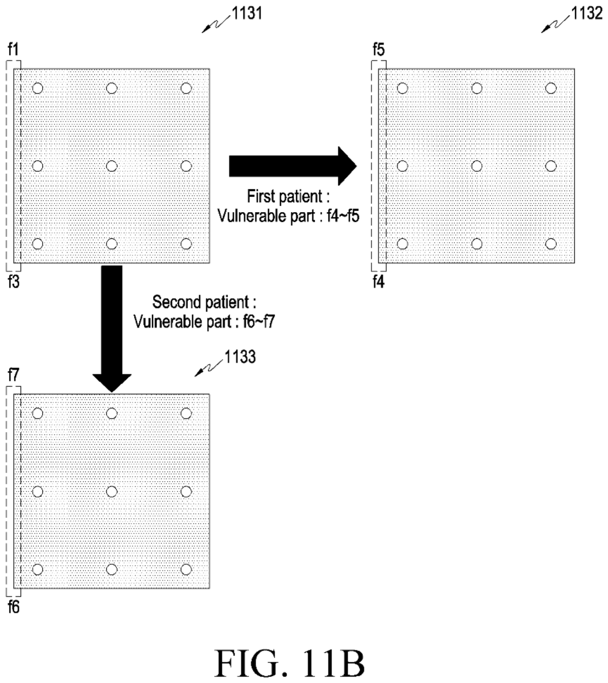

According to an embodiment, the electronic device 100 may provide auditory training content based on information regarding vulnerable points identified for individual users. For instance, as illustrated in Figure 11b, the electronic device 100 may determine that, based on the results of utilizing auditory training content 1131 associated with the first frequency range (f1 to f3), the vulnerable point for a first user lies within the frequency range of f4 to f5, and the vulnerable point for a second user lies within the frequency range of f6 to f7. The electronic device 100 may provide auditory training content 1132 based on the second frequency range (f4 to f5) tailored to the first user's vulnerable point. Similarly, the electronic device 100 may provide auditory training content 1133 based on the third frequency range (f6 to f7) tailored to the second user's vulnerable point. For example, the electronic device 100 may dynamically adjust and provide content corresponding to updated vulnerable points, based on changes in a user's vulnerable areas over time.

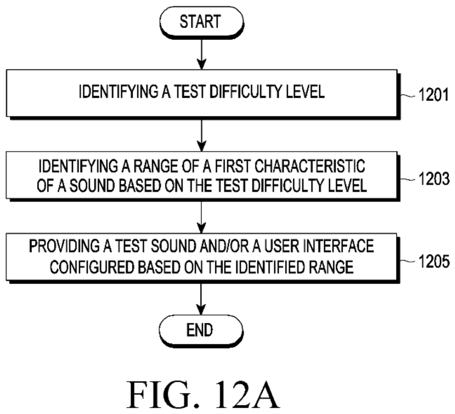

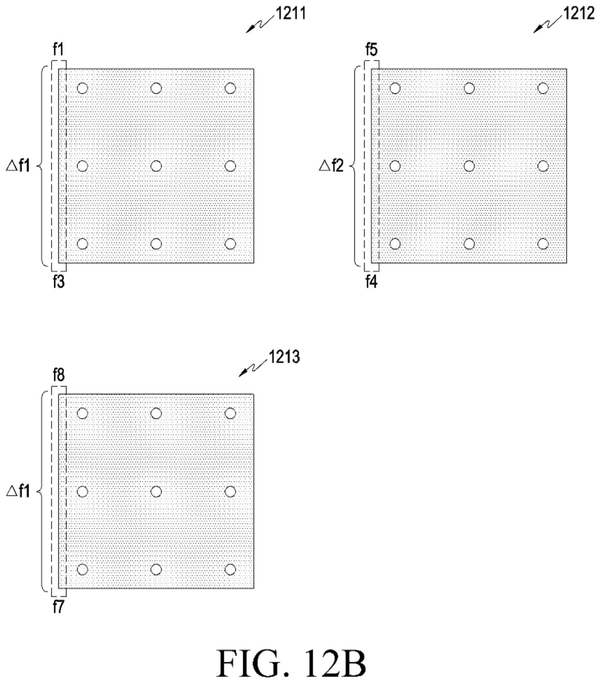

Figure 12a illustrates a diagram explaining a method for providing auditory training content according to an embodiment. The embodiment of Figure 12a is described with reference to Figure 12b, which illustrates user interfaces corresponding to different test difficulty levels.

According to an embodiment, the electronic device 100 may, in operation 1201, determine the test difficulty level. For instance, the test difficulty level may be determined based on settings specified by the user and/or an administrator. Additionally, the test difficulty level may be determined based on the results of previously performed tests, at least in part. For example, the test difficulty level could be adjusted based on the cumulative number of completed tests. However, there are no specific restrictions on the jjs seemaaassaav method of determining the test difficulty level.

The electronic device 100, in operation 1203, may determine the range of a first characteristic of the sound based on the test difficulty level. In operation 1205, the electronic device 100 may provide the test sound and/or user interface configured based on the determined range. For example, referring to Figure 12b, the first user interface 1211 may be represented based on a first frequency range of f1 to f3, with the test sound also designed within the first frequency range of f1 to f3. The size of the first frequency range may be denoted as Δf1. Similarly, the second user interface 1212 may be represented based on a second frequency range of f5 to f6, with the test sound designed within the second frequency range of f5 to f6. The size of the second frequency range may be denoted as Δf2. For instance, a relatively higher test difficulty level may correspond to a smaller range of characteristics in the user interface (e.g., a smaller frequency range). If the test difficulty level of the second user interface 1212 is higher than that of the first user interface 1211, the size of the second frequency range Δf2 may be smaller than the size of the first frequency range Δf1. However, the reverse may also be possible, as understood by those skilled in the art.

In another example, the range of the characteristic, rather than the size of the range, may be set according to the test difficulty level. For instance, the third user interface 1213 may be represented based on a third frequency range of f7 to f8, with the test sound also designed within the third frequency range of f7 to f8. The size of the third frequency range may be denoted as Δf1. This means that the first frequency range and the third frequency range may have the same size, Δf1, but their actual ranges may differ. As the test difficulty level changes, the size of the frequency range may remain constant while the specific frequency range itself is adjusted.

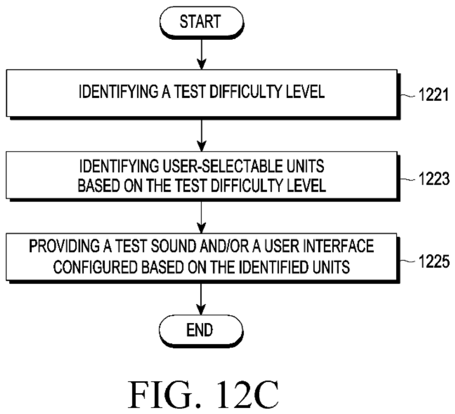

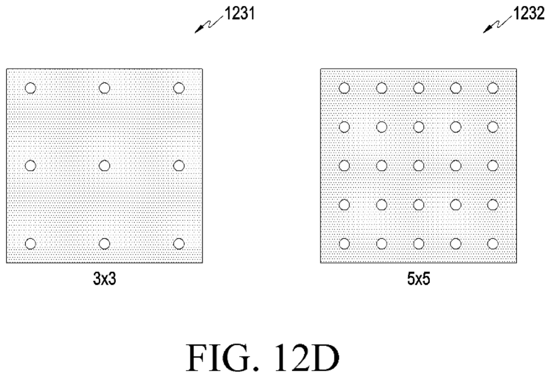

Figure 12c illustrates a method for providing content for auditory training according to an embodiment. The embodiment of Figure 12c is explained with reference to Figure 12d. Figure 12d illustrates user interfaces based on test difficulty levels.

According to one embodiment, the electronic device 100, in operation 1221, may identify the test difficulty level. In operation 1223, the electronic device 100 may determine the selectable units by the user (or expressed as the density and/or number of reference objects) based on the test difficulty level. In operation 1225, the electronic device 100 may provide test sounds and/or user interfaces configured based on the determined units (or the density and/or number of reference objects). For instance, referring to Figure 12d, the first user interface 1231 may include reference objects arranged in a 3x3 grid. The auditory pattern of the test sound corresponding to the first user interface 1231 may be designed based on, for example, three levels corresponding to the vertical number of reference objects. The second user interface 1232 may include reference objects arranged in a 5x5 grid. The auditory pattern of the test sound corresponding to the second user interface 1232 may be designed based on, for example, five levels corresponding to the vertical number of reference objects. For example, as the test difficulty level increases, the selectable units for the user may become relatively smaller, the number of reference objects included in the user interface may become relatively larger, and/or the density of reference objects included in the user interface may become relatively higher.

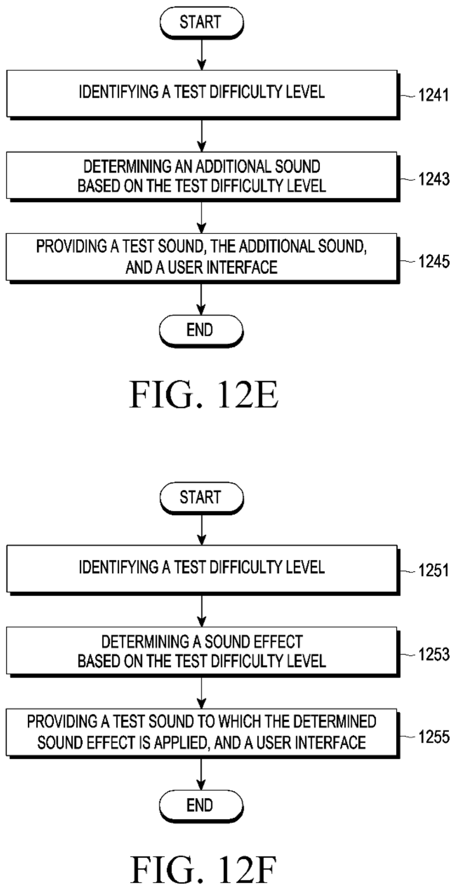

Figure 12e illustrates a method for providing content for auditory training according to an embodiment.

According to one embodiment, the electronic device 100, in operation 1241, may identify the test difficulty level. In operation 1243, the electronic device 100 may determine additional sounds based on the test difficulty level. In operation 1245, the electronic device 100 may provide the test sound, the additional sound, and the user interface. For example, the additional sound may be noise. Listening in an environment with noise can be relatively challenging. Accordingly, based on the test difficulty level, the provision of the additional sound and/or the properties of the additional sound (e.g., type, frequency, timbre, and/or volume of the additional sound, without limitation) may be determined.

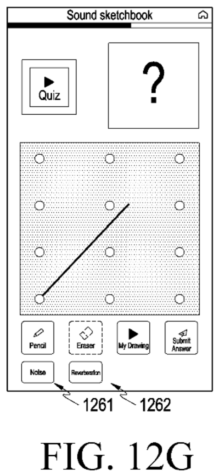

Figure 12f illustrates a method for providing content for auditory training according to an embodiment. The embodiment of Figure 12f is explained with reference to Figure 12g, which describes the application of sound effects according to an embodiment.

According to one embodiment, the electronic device 100, in operation 1251, may identify the test difficulty level. In operation 1253, the electronic device 100 may determine a sound effect based on the test difficulty level. In operation 1255, the electronic device 100 may provide the test sound with the determined sound effect applied, along with the user interface. For example, the sound effect may include a reverberation effect, but there are no limitations on the type or number of sound effects. When a sound effect like reverberation is applied, auditory perception may become relatively challenging. Accordingly, the application of sound effects, their type, number, and/or intensity may be determined based on the test difficulty level. As illustrated in Figure 12g, selectable objects 1261 and 1262 may be provided to the user. For instance, based on the selection of object 1261, the electronic device 100 may provide noise as an additional sound along with the test sound, as described in Figure 12e. Based on the selection of object 1262, the electronic device 100 may provide a test sound with a reverberation effect applied, as described in Figure 12f. For example, when a test sound with a reverberation effect is provided, the electronic device 100 may also visually represent an object corresponding to the user input, with visual effects such as reverberation applied to the object.

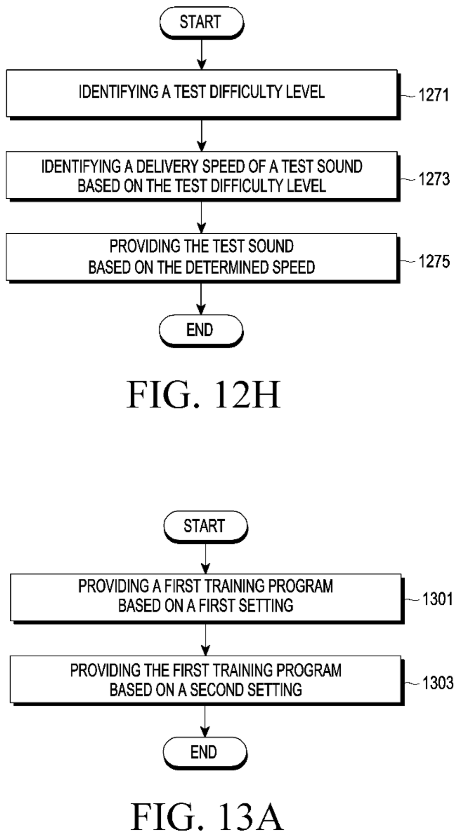

Figure 12h illustrates a method for providing content for auditory training according to an embodiment.

According to one embodiment, the electronic device 100, in operation 1271, identifies the test difficulty level. In operation 1273, the electronic device 100 determines the playback speed for the test sound based on the test difficulty level. In operation 1275, the electronic device 100 provides the test sound at the determined speed. When the playback speed of the test sound is relatively high, auditory perception can become more challenging. Accordingly, the playback speed of the test sound may be determined based on the test difficulty level.

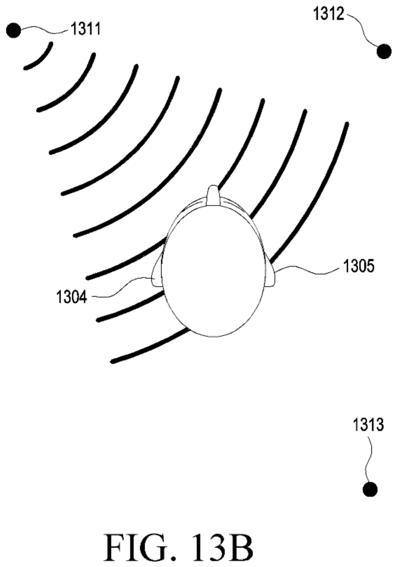

Figure 13a illustrates a method for providing content for auditory training according to an embodiment. The embodiment shown in Figure 13a will be described with reference to Figure 13b, which illustrates the position of a virtual sound source in a stereo environment according to an embodiment.

According to one embodiment, the electronic device 100, in operation 1301, provides a first training program based on a first setting. In operation 1303, the electronic device 100 provides the same first training program based on a second setting. For example, as explained with reference to Figures 12a through 12g, the electronic device 100 may adjust various parameters according to the identified test difficulty levels. For instance, the electronic device 100 may determine parameters corresponding to the first and second settings based on the identified test difficulty levels. In another example, the electronic device 100 may configure the same training program with different voices (or frequencies or timbres).

In one example, the electronic device 100 may provide test sounds for the left ear and right ear in a stereo environment (or an earphone environment). For instance, the electronic device 100 can provide test sounds for the left ear only, the right ear only, or both ears, thereby offering test sounds based on multiple settings. For example, a user may have impaired hearing in the left ear, the right ear, or both ears. The electronic device 100 may configure the combination of test sounds in a stereo environment based on information related to the user's impaired hearing (e.g., cochlear implant surgery details, though not limited to this).

For instance, as shown in Figure 13b, sound output devices 1304 and 1305 (e.g., earphones) may be inserted into the user's ears. By controlling the phases of the sounds outputted from each of the sound output devices 1304 and 1305, the perceived direction of virtual sound sources 1311, 1312, and 1313 can be adjusted. For example, the electronic device 100 can manipulate the phases of the sounds provided by the sound output devices 1304 and 1305 to make the user perceive the test sound as originating from the first virtual sound source 1311, the second virtual sound source 1312, or the third virtual sound source 1313. As described above, the user may have impaired hearing in the left ear, the right ear, or both ears. A user with impaired hearing in the left ear may find it challenging to perceive sounds originating from the left side relative to their position. The electronic device 100 may configure the locations of the virtual sound sources 1311, 1312, and 1313 in various ways based on information about the user's hearing impairment (e.g., cochlear implant surgery details, though not limited to this).

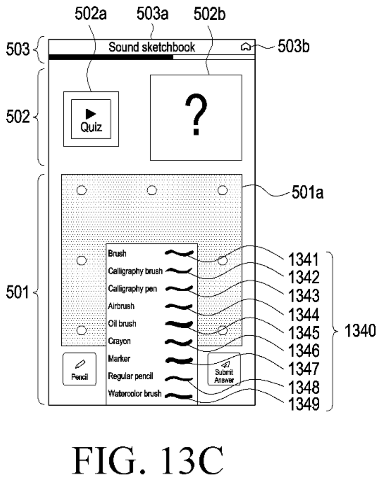

Figure 13c illustrates an exemplary screen for providing content for auditory training according to one embodiment.

According to one embodiment, the electronic device 100 can provide a writing tool list 1340, as shown in Figure 13c. The trigger for displaying the writing tool list 1340 is not limited to any specific condition. The writing tool list 1340 may include multiple objects 1341, 1342, 1343, 1344, 1345, 1346, 1347, 1348, and 1349. Each object (e.g., 1341 through 1349) may visually represent attributes or features corresponding to a specific writing tool (e.g., textual labels and graphical previews of the tool's properties), though this representation is merely exemplary and not restrictive. The electronic device 100 can detect the selection of any object from the writing tool list 1340. Based on the selected object, the device may configure certain properties of the test sound. For example, if the object 1346 labeled as "Crayon" is selected, the electronic device 100 may set the timbre of the test sound to correspond to the attribute associated with the "Crayon" object. For instance, the electronic device 100 might provide a test sound derived from applying a pass filter to white noise. By adjusting the passband width (or Q factor) of the filter, the timbre can be modified. Alternatively, the device may adjust individual components of the test sound (e.g., harmonic elements) to achieve specific timbre changes. The electronic device 100 can be configured to provide test sounds with various attributes corresponding to different objects, enabling a wide range of test sound variations. Furthermore, the device may visually represent the trajectory of a property corresponding to the selected object based on user input. The selection of a writing tool by the user, based on interaction, is merely illustrative. The electronic device 100 may also autonomously select a writing tool based on predefined rules (e.g., difficulty level, user training level, or user vulnerabilities). If a writing tool is selected according to such rules, the electronic device 100 may provide a test sound reflecting the attributes of the selected writing tool. Additionally, the electronic device 100 may visually represent objects reflecting the properties of the selected writing tool. In another example, the electronic device 100 might provide test sounds with predefined attributes and present a user interface requiring the user to identify those attributes. For instance, the user could listen to a test sound with unknown attributes, select the corresponding writing tool from the writing tool list 1340 (as shown in Figure 13c), and input their response. The electronic device 100 can then represent an object with the properties of the user-selected writing tool and provide a sound reflecting the corresponding attributes. The user can compare the sound's properties associated with their selected writing tool to the test sound's properties to determine whether they match. The user may then submit their answer, revise the object, or reselect the writing tool as necessary.

For example, a single test sound may be assigned to a single writing tool; however, this is merely an example. Each of the multiple sub-sounds of a test sound may correspond to different writing tools (e.g., the first part to "Pencil" and the second part to "Crayon"). In this case, selecting different writing tools for each sub-object based on user input may be required. Consequently, the properties of each sub-object could be expressed differently, reflecting the attributes of the corresponding writing tool.

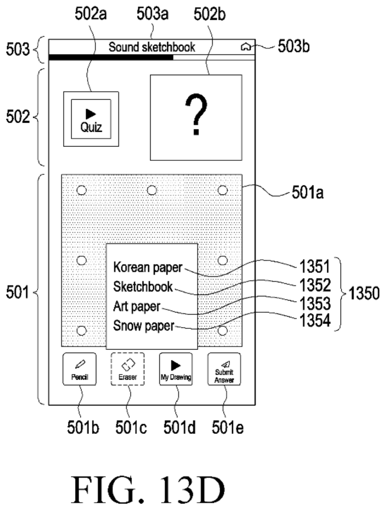

Figure 13d illustrates a screen for providing auditory training content according to an embodiment.

In this embodiment, the electronic device 100 can provide a Paper Material List 1350 as shown in Figure 13d. The trigger for displaying the Paper Material List 1350 is not restricted. The Paper Material List 1350 may include multiple objects 1351, 1352, 1353, 1354. Each object 1351, 1352, 1353, 1354 may be expressed, for example, by including the text name of a paper material; however, this is merely an example and not limiting. The electronic device 100 can identify the selection of any object among the multiple objects 1351, 1352, 1353, 1354 included in the Paper Material List 1350. Based on the selected object, the electronic device 100 can configure the properties of the test sound. For instance, if the object 1351 corresponding to "Korean traditional paper" is selected, the electronic device 100 can set the timbre, a property of the test sound, to correspond to the object 1351. For example, the electronic device 100 can determine the properties of the test sound based on a combination of the writing tool type selected as in Figure 13c and the paper material selected as in Figure 13d. The electronic device 100 can also visually represent the trajectory of the selected object's properties based on user input. For instance, the electronic device 100 can determine the visual object's properties based on the combination of the writing tool type selected as in Figure 13c and the paper material selected as in Figure 13d. The selection of paper material by the user is merely an example. The electronic device 100 may also select a paper material based on predefined rules (e.g., difficulty level, user training level, and user weaknesses) without user input. For instance, if the paper material is selected based on predefined rules, the electronic device 100 can provide test sounds and/or background sounds corresponding to the properties of the selected paper material. Furthermore, the electronic device 100 can visually represent an object with the properties of the selected paper material based on user input. Alternatively, the electronic device 100 can modify the properties of the input area. In another example, the electronic device 100 can provide a test sound with predefined properties based on specified rules and offer a user interface requiring the user to select these properties. For instance, the user can listen to a test sound and/or background sound with unknown properties, select a corresponding paper material through the Paper Material List 1350 as in Figure 13d, and then input their response. The electronic device 100 can visually represent an object with the properties of the paper material selected by the user and/or provide a sound with the properties of the paper material selected by the user. The user can then determine whether the sound properties of their selected paper material match the test sound and/or background sound properties. If they do not match, the user can submit a correction, such as reselecting the paper material.

In Figures 13c and/or 13d, while examples are provided where the properties of the test sound are configured based on the writing tool and/or paper material, these are merely illustrative. For instance, the electronic device 100 may also configure the properties of additional sounds (e.g., background sounds) provided along with the test sound based on at least a part of the writing tool or paper material. For example, the properties of the test sound may be adjusted based on the writing tool, while the properties of the additional sound (e.g., background sound) may be adjusted based on the paper material. However, this is merely illustrative and not limiting.

For instance, one paper material may be assigned to a test sound and/or background sound, but this is just an example. It is also possible for multiple sub-sounds of a test sound and/or background sound to correspond to different paper materials (e.g., the first part corresponds to sketchbook paper, and the second part corresponds to Korean traditional paper). In such cases, the selection of different paper materials for each sub-object associated with user input may be required. Consequently, the properties of each sub-object and/or the corresponding background may be distinctly represented.

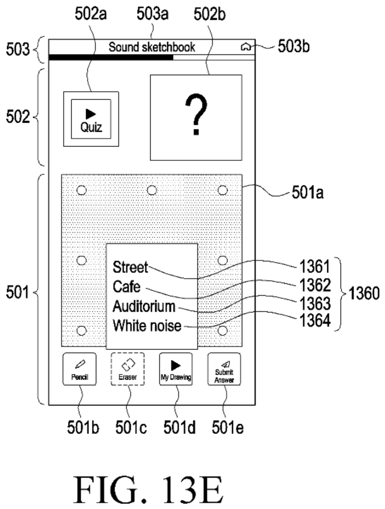

Figure 13e illustrates a screen for providing auditory training content according to an embodiment.

According to one embodiment, the electronic device 100 may provide a background sound list 1360 as shown in Figure 13e, with no limitations on the trigger for providing the list. The background sound list 1360 may include multiple objects 1361, 1362, 1363, and 1364. Each object may be represented, for example, with text indicating the name of the background sound, though this is merely illustrative and not limiting. The electronic device 100 may confirm the selection of any one of the objects from the background sound list 1360. Based on the selected object, the electronic device 100 may configure the properties of the background sound. For instance, if the object 1361 corresponding to "street" is selected, the electronic device 100 may set a street ambiance as the background sound. The selection of the background sound by the user is merely an example. The electronic device 100 may also select a background sound without user input, based on predefined rules (e.g., difficulty level, user training level, and/or user weaknesses). When the background sound is selected based on such rules, the electronic device 100 may provide a background sound with properties corresponding to the selected background sound. In another example, the electronic device 100 may provide a background sound with predefined properties and require the user to select the background sound's attributes via a user interface. For instance, the user may listen to an unknown background sound combined with the test sound and select the corresponding background sound's properties using a background sound list like the one shown in Figure 13e, followed by entering user input. The electronic device 100 may display the object selected by the user and/or provide the background sound with the selected properties along with the user-input-based sound. The user may determine whether the properties of the background sound they selected match those of the background sound provided with the test sound. They can submit a response or modify their selection by re-selecting a background sound object. For example, one background sound may be assigned a single property, but this is merely illustrative. It is also possible for multiple sub-sounds of a background sound to correspond to different properties (e.g., the first part corresponds to a "street," and the second part corresponds to a "hall"). In this case, different sub-objects corresponding to user input may require selecting different background sounds. Additionally, each region may be represented with different background images corresponding to different background sounds (e.g., the first part with a street image and the second part with a hall image). If the auditory training content is presented in a VR environment, visual content may be provided to represent virtual reality corresponding to the selected background sound.

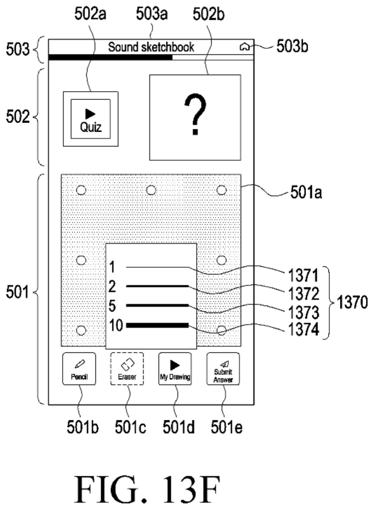

Figure 13f illustrates a screen for providing auditory training content according to an embodiment.

According to one embodiment, the electronic device 100 may provide a writing tool thickness list 1370, as shown in Figure 13f, with no limitations on the trigger for its display. The writing tool thickness list 1370 may include multiple objects 1371, 1372, 1373, and 1374. Each object may represent, for example, text indicating the thickness of the writing tool and/or an example of an object property corresponding to the thickness. However, this representation is merely illustrative and not limiting. The electronic device 100 may identify the selection of any one of the objects in the writing tool thickness list 1370. Based on the selected object, the electronic device 100 may configure the properties of the test sound. For instance, if the object 1372 corresponding to a "thickness of 2" is selected, the electronic device 100 may set the volume of the test sound, as one of its properties, to the volume corresponding to object 1372. The electronic device 100 may also represent an object showing the trajectory of the selected thickness based on user input. The selection of the writing tool's thickness by the user is merely an example. The electronic device 100 may select the thickness without user input, based on predefined rules (e.g., difficulty level, user training level, and/or user weaknesses). If the thickness of the writing tool is selected based on such rules, the electronic device 100 may provide a test sound with properties corresponding to the selected thickness. Additionally, the electronic device 100 may represent an object with the selected thickness based on user input. In another example, the electronic device 100 may provide a test sound with predefined properties (e.g., volume) and require the user to select the corresponding properties via a user interface. For instance, the user may listen to a test sound with unknown properties, select the corresponding thickness using the writing tool thickness list 1370, and then provide user input. The electronic device 100 may display an object representing the selected thickness and/or provide a sound with the selected thickness property. The user can determine whether the sound property corresponding to the selected thickness matches the test sound property. The user may then submit their response or modify the selection, such as reselecting a thickness from the writing tool list. The setting of thickness through the writing tool list 1370 is merely illustrative. The electronic device 101 may also set the thickness of the object based on factors such as pressure intensity and/or touch duration (e.g., in proportion). For instance, a single test sound may be assigned one thickness, but this is merely illustrative. It is also possible for multiple sub-sounds within the test sound to correspond to different thicknesses (e.g., the first part corresponds to "thickness of 2," and the second part corresponds to "thickness of 5"). In such cases, selecting different thicknesses for each sub-object corresponding to user input may be required. As a result, the properties of each sub-object (e.g., thickness) may be represented differently.

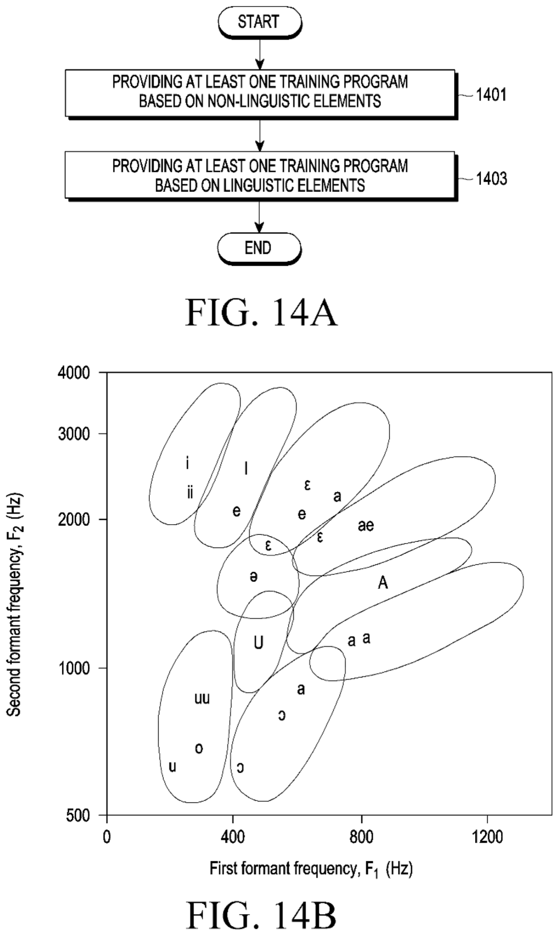

Figure 14a illustrates a method for providing auditory training content according to an embodiment. Figure 14a will be explained with reference to Figure 14b, which depicts an analysis based on formants according to an embodiment.

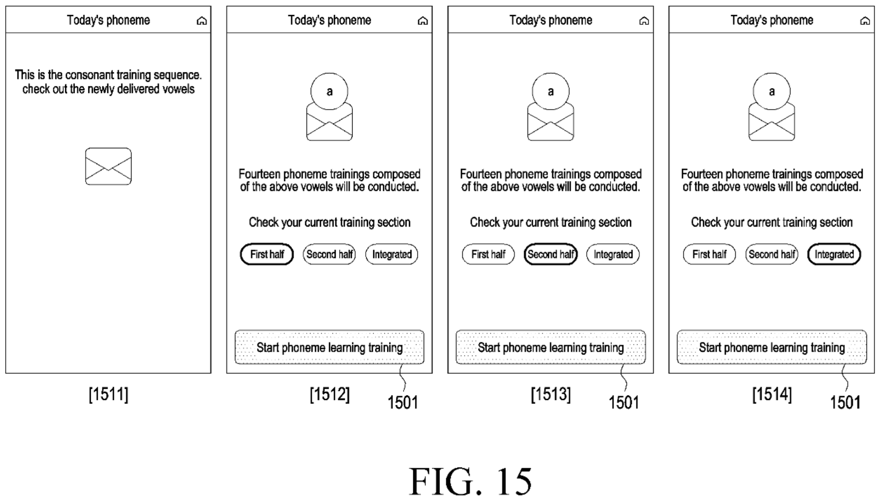

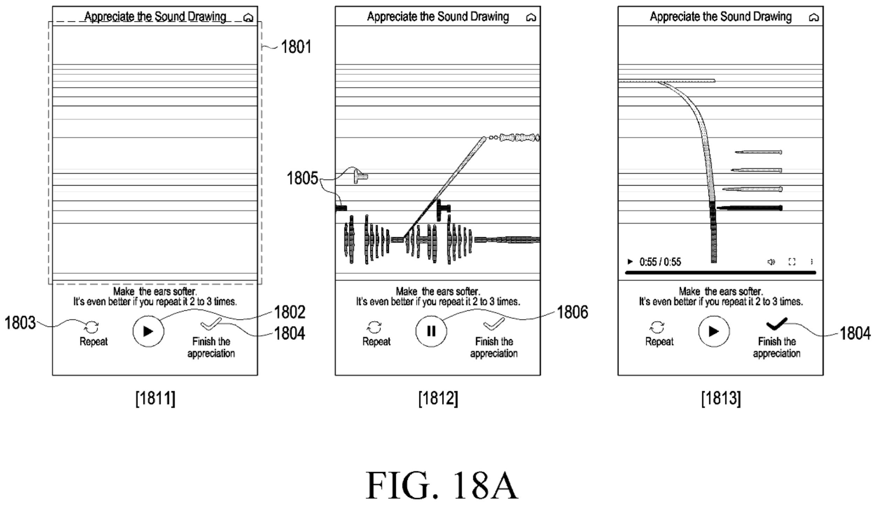

According to one embodiment, the electronic device 100 may, in operation 1401, provide at least one training program based on non-linguistic elements. For instance, the electronic device 100 may provide a training program based on the matching of auditory patterns and visual patterns, as explained with reference to Figures 5a through 5i, as at least one training program based on non-linguistic elements. Additionally, the electronic device 100 may provide a training program based on the matching of auditory patterns and visual patterns, which will be further detailed with reference to Figures 18a through 18c, as at least one training program based on non-linguistic elements. In operation 1403, after providing at least one training program based on non-linguistic elements, the electronic device 100 may provide at least one training program based on linguistic elements. A training program based on linguistic elements may be associated with phonemes defined by language, which will be described in greater detail with reference to Figures 15a through 17. It is worth noting that providing a training program based on linguistic elements after providing one based on non-linguistic elements is merely illustrative; the sequence of provision may vary. Additionally, at least some parts of the training program based on phonemes defined by language may be included in the training program based on non-linguistic elements. For instance, as shown in Figure 14b, pronunciations of phonemes defined by language may be positioned on a coordinate plane based on the first formant frequency and the second formant frequency. Accordingly, a relatively high number of incorrect responses in a test for a specific phoneme may indicate that the user is less proficient within the corresponding frequency range. Based on this information, the electronic device 100 may set additional training programs tailored to address the identified weakness in that frequency range.

Figure 15 illustrates content for auditory training according to an embodiment.

According to one embodiment, the electronic device 100 may provide screens 1511, 1512, 1513, and 1514 associated with phoneme training information. Screen 1511 may indicate the start of training for a new phoneme. Screen 1512 may include information regarding the target vowel (e.g., " 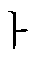 ", the Korean vowel). The phoneme training may be configured to proceed partially (e.g., in sections such as the first half and second half, though not limited to this) or integratively for phonemes (e.g., 14 phonemes) based on the target vowel, as illustrated in screens 1512, 1513, and 1514. For instance, when the training start object 1501 within screens 1512, 1513, and 1514 is selected, the electronic device 100 may provide a first self-assessment training screen. In one example, the electronic device 100 may select a target vowel and determine the training phoneme by combining the selected vowel with multiple consonants. The target vowel may be selected based on the user's training performance history, cumulative training count, or test difficulty, though the selection criteria are not limited to these. In another example, the electronic device 100 may be implemented to select a target consonant and determine the training phoneme by combining it with multiple vowels.

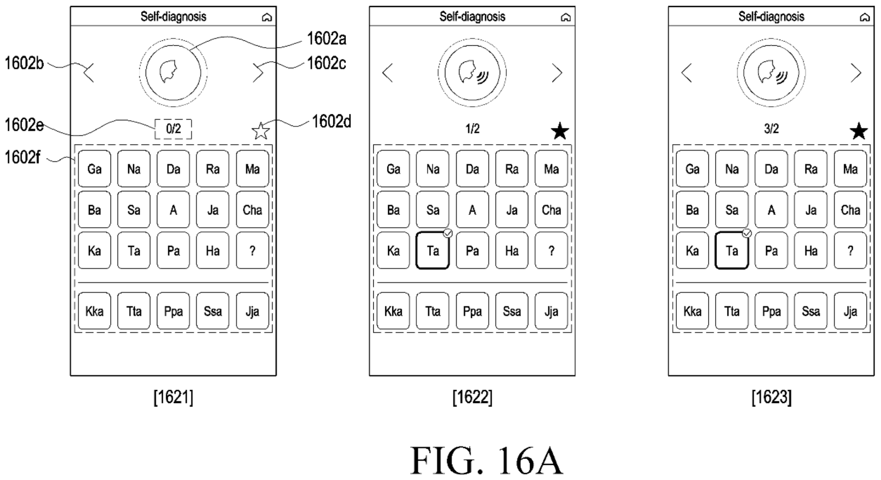

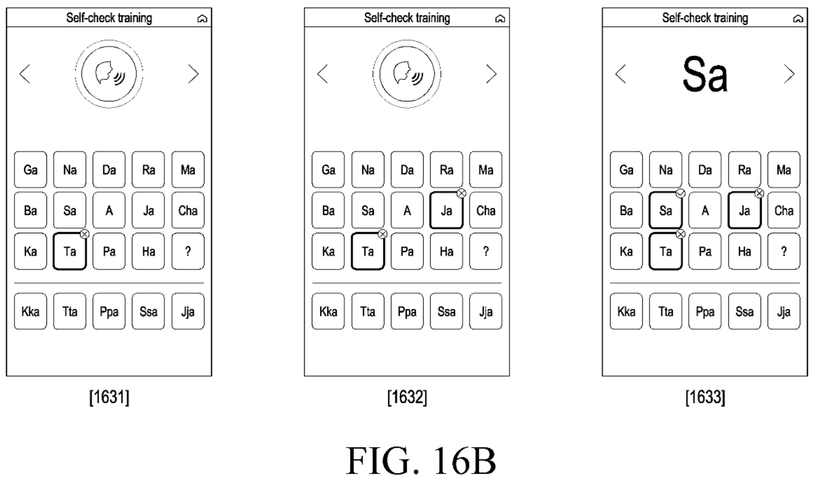

Figures 16a and 16b are diagrams for explaining content for auditory training according to an embodiment.

According to one embodiment, the electronic device 100 may provide at least some of the screens 1621, 1622, 1623, 1631, 1632, and 1633, as shown in Figures 16a and 16b, based on the selection of the training start object 1501 in Figure 15. For example, as shown in Figures 16a and 16b, a third playback object 1602a for requesting playback of a question (e.g., one of the syllables combining a consonant and a vowel to be trained), a first navigation object 1602b for moving to the previous question, a second navigation object 1602c for moving to the next question, and a registration object 1602d for registering a question of interest may be provided. For example, the first self-assessment training screen may include a count display area 1602e for displaying the listening count for each question and a syllable display area 1602f for displaying multiple selectable syllables.

For example, the training may proceed in a manner where the user listens to the provided question and selects one of the multiple syllables displayed in the syllable display area 1602f, but this is not limited thereto. For example, when the third playback object 1602a is selected, the electronic device 100 may provide the corresponding syllable through an audio output device. Referring to screen 1622, it can be confirmed that one of the syllables displayed in the syllable display area 1602f has been selected by the user. Referring to screens 1622 and 1623, the listening count in the count display area 1602e may increase each time the third playback object 1602a is selected.

For example, referring to screens 1631 and 1632, when an incorrect answer is input, it may be visually notified that the answer is incorrect (e.g., changing the background to a first color, such as red, and/or displaying a designated symbol in the first color, such as "x," on one side). For example, referring to screen 1633, when the correct answer is selected, it may be visually notified that the answer is correct (e.g., changing the background to a second color, such as green, displaying a designated symbol in the second color, such as "√," on one side, and/or replacing the third playback object 1602a with the correct syllable).

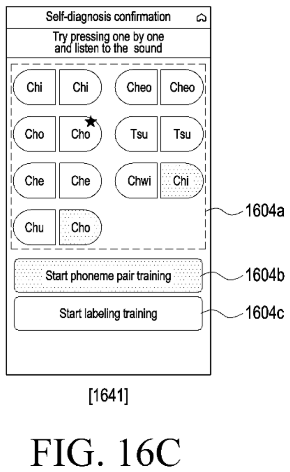

For example, after completing the training up to the last question, when the second movement object 1602c to request the next question is selected, a first self-diagnosis result screen may be provided. As shown in FIG. 16c, the first self-diagnosis result screen 1641 may include a result display area 1604a that displays the syllable (or character) corresponding to the question and the syllable selected by the user as a pair (hereinafter referred to as a phoneme pair). For example, the syllable corresponding to the question may be displayed on the left, and the syllable selected by the user may be displayed on the right, thereby displaying a single phoneme pair. For example, when an incorrect answer is confirmed, other syllables may be displayed with a different background color.

For example, the first self-diagnosis result screen 1641 may include a phoneme pair training object 1604b and a labeling training object 1604c for incorrect questions. At this time, the labeling training object 1604c may be displayed in a deactivated state and activated only after the phoneme pair training is completed. For example, the phoneme pair training object 1604b may be deactivated (or not displayed) if there were no incorrect answers in the previous training, and only the labeling training object 1604c may be activated.

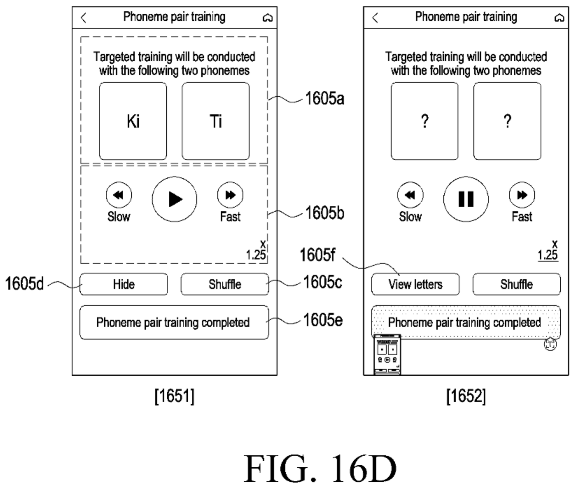

For example, when the phoneme pair training object 1604b is selected, a phoneme pair training screen may be provided. Phoneme pair training involves repeatedly listening to the incorrect phoneme pair to help the user recognize the differences. For instance, when the phoneme pair training object 1604b is selected, as shown in screen 1651 of FIG. 16d, the phoneme pair training screen may be provided. The phoneme pair training screen may include a phoneme pair display area 1605a that provides information about the phoneme pair to be trained, a playback control area 1605b to control playback speed, a sequence shuffle object 1605c to randomly change the order of the phoneme pairs, a hide object 1605d, and/or a training end object 1605e.

After a designated period of time has elapsed, as shown in screen 1652 of FIG. 16d, the phoneme pair to be trained may be hidden (e.g., displayed as a question mark), and the order of the phoneme pair may be randomly changed so that the sounds corresponding to each phoneme are alternately provided. At this point, the hide object 1605d may be replaced with a letter display object 1605f, which reveals the hidden letters. After sufficiently listening to the alternately provided phoneme pair, the user can select the letter display object 1605f to confirm whether they have accurately perceived the sounds. At this time, the letter display object 1605f may be replaced back with the hide object 1605d.

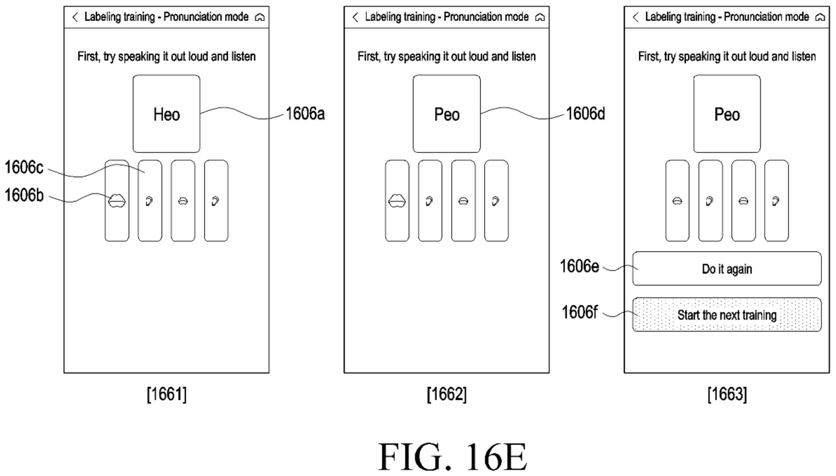

For example, when the phoneme pair training is completed, the labeling training object 1604c may be activated. Upon selection of the labeling training object 1604c, the labeling training screen may be provided. The labeling training may include articulation training and self-diagnostic training. The articulation training involves repeating speaking (or pronouncing) and listening (or hearing). For instance, as shown in screen 1661 of FIG. 16e, the syllable to be trained (e.g., " 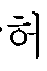 ", the Korean which is pronounced as "Hu") 1606a may be displayed on one side, and the speaking icon 1606b and the listening icon 1606c may be alternately activated. When the listening icon 1606c is activated, the sound of the corresponding syllable may be provided. According to some embodiments, the user's voice may also be recorded when the speaking icon 1606b is activated.

For example, after the specified number of repetitions (e.g., two times) for the articulation training has elapsed, the training screen for the next syllable (e.g., " 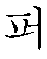 ", the Korean which is pronounced as "Pu") 1606d may be provided, as shown in screen 1662. Once all training is completed, the articulation training result screen may be displayed, as shown in screen 1663. The articulation training result screen may include a relearning object 1606e for retraining the articulation training and/or a next training object 1606e to end the articulation training and proceed to the next training.

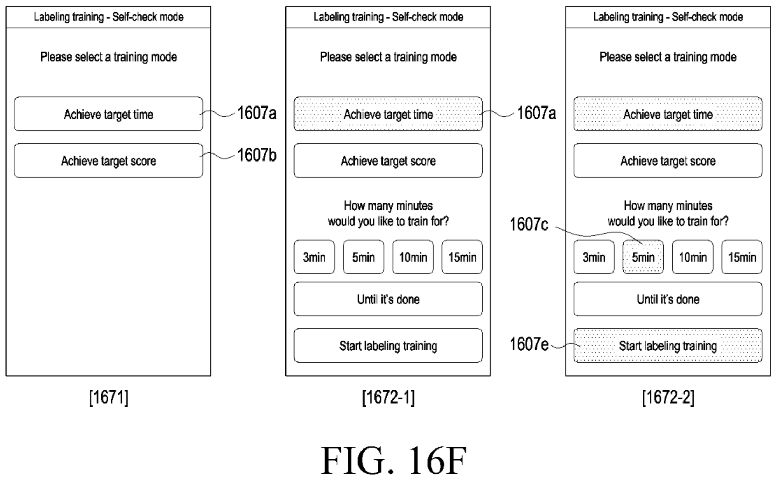

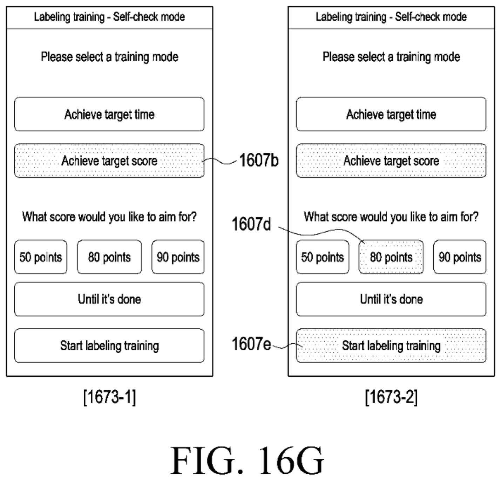

For example, when the next training object 1606e is selected, self-assessment training for the labeling training may be provided. For instance, as shown in 1671 of Figure 16f, a screen for selecting a training mode of the self-assessment training in labeling training may be provided. The self-assessment training in labeling training may include a target time training mode 1607a, where training is performed for a target time, and a target score training mode 1607b, where training is performed until a target score is achieved. Once either mode is selected by the user, the target is set, and the self-assessment training in labeling training may begin. For example, as shown in screens 672-1 and 672-2, the target time training mode 1607a may be selected, and a target time (e.g., 5 minutes) 1607c may be set. In another example, as shown in screens 1673-1 and 1673-2 of Figure 16g, the target score training mode 1607b may be selected, and a target score (e.g., 80 points) 1607d may be set. When the target time 1607c or target score 1607d is set, a training start object 1607e may be activated.

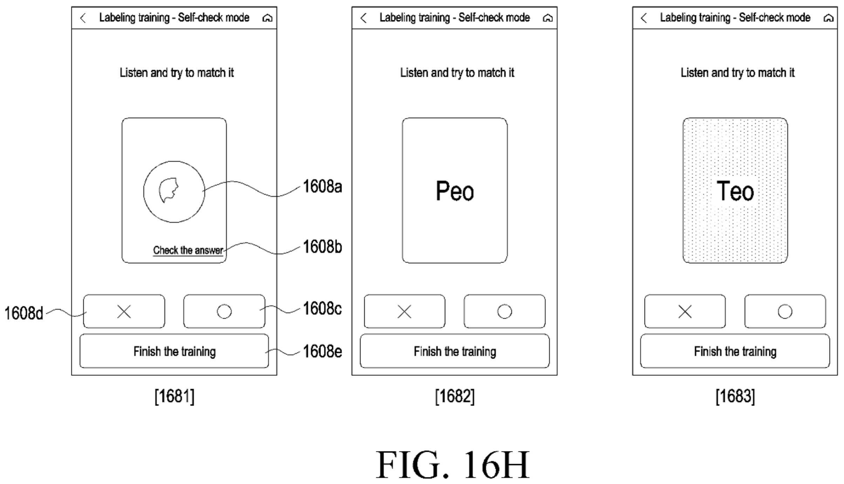

Once the target time 1607c or target score 1607d is set and the training start object 1607e is selected, a self-assessment training screen 1681 for labeling training may be provided, as shown in Figure 16h. The self-assessment training screen 1681 for labeling training may include a fourth playback object 1608a, an answer view object 1608b, a correct answer object 1608c, an incorrect answer object 1608d, and/or a training end object 1608e. When the fourth playback object 1608a is selected, a sound corresponding to the trained syllable may be provided. The user can estimate the syllable of the listened sound. When the answer view object 1608b is selected, as shown in screen 1682, the fourth playback object 1608a may be changed (or replaced) with the syllable (or letter) corresponding to the sound. For example, the electronic device 100 may activate the correct answer object 1608c and the incorrect answer object 1608d, which were in a deactivated state, so that the user can select them.

The user can compare the syllable they estimated with the correct answer and select one of the correct answer object 1608c or the incorrect answer object 1608d. For example, if the user's estimated syllable matches the correct answer, they may select the correct answer object 1608c, and if it does not match, they may select the incorrect answer object 1608d. Once either the correct answer object 1608c or the incorrect answer object 1608d is selected, the next question screen may be provided. Specifically, if the correct answer object 1608c is selected, as shown in screen 1683, the corresponding sound may be provided once, and a visual notification indicating correctness (e.g., changing the border of the area displaying the syllable to a first color) may be displayed before the next question screen is provided. Conversely, if the incorrect answer object 1608d is selected, the corresponding sound may be provided a designated number of times (e.g., three times), and a visual notification indicating the incorrectness (e.g., blinking the border of the area displaying the syllable in a second color) may be displayed before the next question screen is provided.

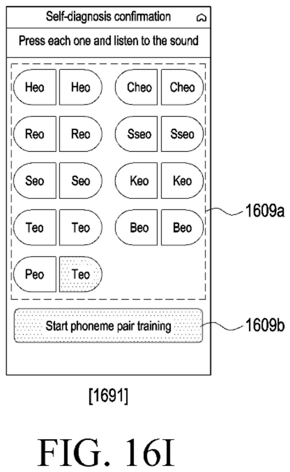

If the target time 1607c or target score 1607d is reached, the electronic device 100 may activate the training end object 1608e, which was previously in a deactivated state (allowing it to be selectable by the user). Even if the target time 1607c or target score 1607d is reached, the user may choose not to select the training end object 1608e and continue with additional training. Meanwhile, when the training end object 1608e is selected, the electronic device 100 may provide the result screen 1691, as shown in Figure 16i. The result screen 1691 may include a result display area 1609a that displays the trained phoneme pairs, similar to the first self-diagnosis result screen 1641 in Figure 16c. However, since both the phoneme pair training and labeling training have been completed, the result screen 1691 may include the next training object 1609b (e.g., for a second self-diagnosis training) instead of the phoneme pair training object 1604b and the labeling training object 1604c.

When the next training object 1609b is selected, the electronic device 100 may provide a second self-diagnosis training. According to some embodiments, when the next training object 1609b is selected, the electronic device 100 may display a home screen. The electronic device 100 may display the object corresponding to the completed training in an active state.

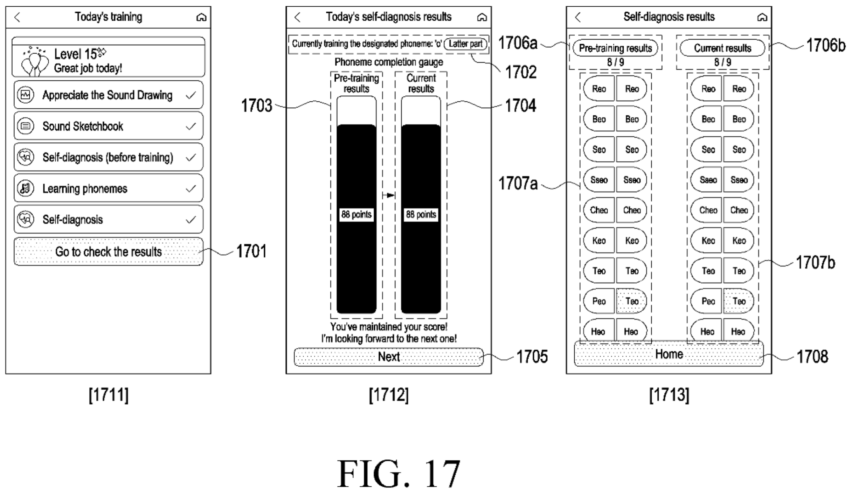

Referring to Figure 17, when the second self-diagnosis training is completed, the electronic device 100 may provide (or return to) a sub-home screen 1711. On the sub-home screen 1711 of Figure 17, objects corresponding to all completed trainings may be displayed in an active state. Additionally, the sub-home screen 1711 of Figure 17 may include a result check object 1701. The result check object 1701 may be displayed based on the completion of all the trainings represented on the sub-home screen 1711.

When the result check object 1701 is selected, the electronic device 100 may provide a first result screen 1712. The first result screen 1712 may include phoneme information 1702 being trained, the first self-diagnosis training result 1703, the second self-diagnosis training result 1704, and a next object 1705 for requesting a second result screen. On the first result screen, the first self-diagnosis training result 1703 and the second self-diagnosis training result 1704 may be provided, for example, using a bar graph, although this is not limited thereto.

When the next object 1705 is selected, the electronic device 100 may provide a second result screen 1713. The second result screen 1713 may include the accuracy rate 1706a of the first self-diagnosis training, the accuracy rate 1706b of the second self-diagnosis training, the phoneme pairs 1707a trained in the first self-diagnosis training, the phoneme pairs 1707b trained in the second self-diagnosis training, and a home object 1708 for returning to the home screen.

FIG. 18a is a diagram illustrating content for auditory training according to an embodiment.

Referring to FIG. 18a, the electronic device 100 may provide screens 1811, 1812, and 1813 for sound picture appreciation training. The provision of content for sound picture appreciation training may include the provision of sounds with auditory patterns where features (e.g., frequency, timbre, etc., but not limited thereto) change or are maintained over time and the provision of objects with visual patterns visualizing the auditory patterns of the features of the output sounds.

For example, when sound picture appreciation training is requested, the electronic device 100 may provide a sound picture appreciation training screen 1811. The sound picture appreciation training screen 1811 may include a visual area 1801 for displaying visual objects, a fifth playback object 1802 for playing a designated training sound, a repeat object 1803 for replaying the training sound, and a finish appreciation object 1804 for ending the sound picture appreciation training.

When the fifth playback object 1802 is selected, the electronic device 100 may provide (e.g., play or output) the designated training sound. Screen 1812 may include a visual object 1805 corresponding to the designated training sound, displayed in the visual area 1801. At this time, the fifth playback object 1802 may be changed to a stop object 1806. The presentation of the visual object 1805 may be substantially synchronized with the provision of the training sound. For example, as the training sound is provided over time, an animation effect may be applied so that the visual object 1805 is gradually displayed. However, it will be understood by those skilled in the art that the synchronization method is not limited thereto.

When the playback of the training sound is completed, the electronic device 100 may activate the finish appreciation object 1804. At this time, the stop object 1806 may be changed (or restored) to the fifth playback object 1802. Subsequently, the user can select the finish appreciation object 1804 to end the sound picture appreciation training, select the fifth playback object 1802 to replay the sound picture appreciation training, or select the repeat object 1803 to repeat the training a designated number of times (e.g., 5 times) or until the stop object 1806 is selected.

While listening to the sound with auditory patterns, the user can view the object with visual patterns, enabling them to visually comprehend the auditory pattern and map it to their auditory results. This allows the user's hearing to be effectively trained.

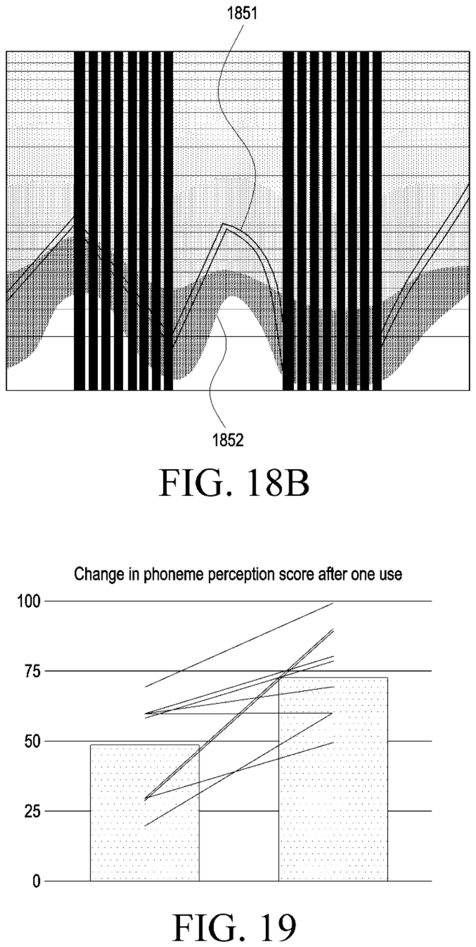

FIG. 18b illustrates content for auditory training according to an embodiment.

According to an embodiment, the electronic device 100 may provide designated effects and/or background sounds along with the training sound. For example, the electronic device 100 may display an object 1851 with a visual pattern corresponding to the training sound, substantially synchronized with the training sound. The electronic device 100 may display an object 1852 corresponding to a designated effect (e.g., a disperse effect). Although not shown, for intermittent pauses during playback, the electronic device 100 may display an object corresponding to a background sound. By checking not only the training sound but also the visual objects corresponding to the applied effects and/or background sounds, the user may undergo auditory training with increased difficulty.

FIG. 19 illustrates changes in auditory evaluation according to the performance of auditory training content in an embodiment

Referring to FIG. 19, the effects of performing auditory training content according to the embodiment can be reviewed.

### 1. Experimental Design

Eight adult participants who had undergone cochlear implant surgery were selected. The participants were instructed to 1) perform an auditory perception evaluation before engaging in the auditory training content according to the embodiment, and 2) perform the auditory perception evaluation again after completing the auditory training content, with the results compared accordingly.

Specifically, the procedure followed the order of 1) pre-evaluation, 2) performing the content according to the embodiment, and 3) post-evaluation. The content based on the embodiment was carried out for a duration of 30 to 50 minutes.

### 2. Evaluation Method

A Korean consonant and vowel discrimination test was conducted. Participants listened to consonants such as ga, na, da, ra, ..., ka, and kka, and the correctness of the participants' recognition results was evaluated.

### 3. Results

Referring to Figure 19, it can be observed that the average accuracy rate before using the content was 48%, whereas the average accuracy rate after using the content increased to 73%. Furthermore, a significant improvement in accuracy rate was observed in 7 out of 8 participants, with the exception of one participant.

As described above with reference to the illustrated embodiments of the present invention, these are merely examples, and it will be apparent to those skilled in the art to which the present invention pertains that various modifications, changes, and equivalent embodiments can be made without departing from the gist and scope of the present invention. Therefore, the true technical protection scope of the present invention shall be determined by the technical spirit of the appended claims.

## Claims

1. A method for auditory training, the method comprising:
providing a test sound having a first auditory pattern for at least one characteristic of a sound;
based on a user input that is input through a user interface for receiving a visual pattern, providing, through the user interface, a first visual object having a first visual pattern corresponding to the user input, wherein a first sound, which is substantially synchronized with a detection time point of the user input and has at least one characteristic identified based on position of the user input defined in the user interface, is provided; and
based on identifying an event for providing a second visual pattern corresponding to the test sound, providing a second visual object having the second visual pattern corresponding to at least one characteristic of the first auditory pattern.

2. The method of claim 1,
wherein providing the first visual object through the user interface comprises:
based on detecting at least a part of the user input associated with a first position and a second position of the user interface, providing at least a part of the first visual object associated with the first position, the second position, and at least one intermediate position between the first position and the second position of the user interface.

3. The method of claim 2,
wherein based on at least a part of the user input being associated with the first position, a first portion of the first sound having at least one characteristic corresponding to the first position is provided;
based on at least a part of the user input being associated with each of the at least one intermediate position, at least one intermediate portion of the first sound having at least one characteristic corresponding to each intermediate position is provided;
and based on at least a part of the user input being associated with the second position, a second portion of the first sound having at least one characteristic corresponding to the second position is provided.

4. The method of claim 2,
wherein at least a part of the user input comprises an input for designating the first position and the second position, and/or an input for designating the first position, the at least one intermediate position, and the second position.

5. The method of claim 1,
wherein the test sound comprises a plurality of portions provided sequentially over time,
and each of the plurality of portions has at least one characteristic that changes or remains constant over time according to the first auditory pattern.

6. The method of claim 1, further comprising:
providing a result of comparing the first visual object and the second visual object.

7. The method of claim 6, wherein the providing the comparison result comprises:
providing information about a user's vulnerable points identified based on the comparison result.

8. The method of claim 1,
wherein the event for providing the second visual pattern corresponding to the test sound comprises:
a selection for affordance to provide a correct visual object, completion of providing the first visual object, and/or passage of a specified time.

9. The method of claim 1, further comprising:
after the first visual object is provided and before the second visual object is provided, performing modification of at least a part of the first visual object based on another user input for modifying the first visual object.

10. The method of claim 9, wherein performing the modification of at least a part of the first visual object comprises:
identifying a deletion command for a first portion of the first visual object; and
deleting the first portion associated with the deletion command while maintaining display of remaining portion of the first visual object excluding the first portion.

11. The method of claim 1,
wherein at a specific time point, each of at least one characteristic of at least a portion of the test sound has a single value.

12. The method of claim 1,
wherein at a specific time point, each of at least one characteristic of at least a portion of the test sound has multiple values.

13. The method of claim 1,
wherein the user interface comprises a plurality of reference objects for association with the user input.

14. The method of claim 13,
wherein the plurality of reference objects are arranged in a grid,
and the user input comprises an input connecting one of a plurality of first reference objects included in one of the columns to one of a plurality of second reference objects included in an adjacent column.

15. The method of claim 13,
wherein based on two or more of the reference objects being associated with a first temporary user input, a visual object associated with the two or more reference objects is provided as the first visual object;
and based on two or more of the reference objects not being associated with a second temporary user input, a visual object temporarily provided based on a trajectory of the second temporary user input is discontinued.

16. The method of claim 13,
wherein the number, density, and/or arrangement of the plurality of reference objects is set based on user selection and/or test difficulty.

17. The method of claim 1,
wherein the first auditory pattern of the test sound is represented based on at least one quantifiable characteristic,
and a lower limit value for the test sound, an upper limit value for the test sound, and/or a difference between the upper and lower limit values is set based on user selection and/or test difficulty.

18. The method of claim 1,
wherein a sound effect identified based on user selection and/or test difficulty is applied to at least a part of the test sound.

19. The method of claim 1, further comprising:
providing background sound identified based on user selection and/or test difficulty along with at least a part of the test sound during the provision of the test sound.

20. The method of claim 1, further comprising, before providing the test sound:
setting at least one different characteristic of a sound other than the at least one characteristic of the first auditory pattern based on user selection and/or test difficulty.

21. The method of claim 1, further comprising:
providing at least one indicator visually notifying playback point of the test sound over time along with the provision of the test sound.

22. A system for auditory training, comprising:
a server; and
an electronic device including at least one processor,
wherein the server is configured to provide instructions to the electronic device based on a connection to the server by the electronic device and/or a request to the server,
and wherein the instructions, when executed based on at least a portion of the at least one processor of the electronic device, cause the electronic device to perform at least one operation, and the at least one operation comprises:
providing a test sound having a first auditory pattern for at least one characteristic of a sound;
based on a user input that is input through a user interface for receiving a visual pattern, providing, through the user interface, a first visual object having a first visual pattern corresponding to the user input, wherein a first sound, which is substantially synchronized with a detection time point of the user input and has at least one characteristic identified based on the position of the user input defined in the user interface, is provided; and
based on identifying an event for providing a second visual pattern corresponding to the test sound, providing a second visual object having the second visual pattern corresponding to at least one characteristic of the first auditory pattern of the test sound.

23. The system of claim 22,
wherein the operation of providing the first visual object through the user interface comprises: based on detecting at least a part of the user input associated with a first position and a second position of the user interface, providing at least a part of the first visual object associated with the first position, the second position, and at least one intermediate position between the first and second positions of the user interface.

24. The system of claim 23,
wherein based on at least a part of the user input being associated with the first position, a first portion of the first sound having at least one characteristic corresponding to the first position is provided;
based on at least a part of the user input being associated with each of the at least one intermediate position, at least one intermediate portion of the first sound having at least one characteristic corresponding to each intermediate position is provided;
and based on at least a part of the user input being associated with the second position, a second portion of the first sound having at least one characteristic corresponding to the second position is provided.

25. The system of claim 23,
wherein at least a part of the user input comprises an input for designating the first position and the second position, and/or an input for designating the first position, the at least one intermediate position, and the second position.

26. A method for auditory training by a system including a server and an electronic device, comprising:
providing, by the server, instructions to the electronic device based on a connection to the server by the electronic device and/or a request to the server; and
executing, by the electronic device, the instructions, wherein the instructions, when executed based on at least a portion of at least one processor of the electronic device, cause the electronic device to perform at least one operation,
and the at least one operation comprises:
providing a test sound having a first auditory pattern for at least one characteristic of a sound; based on a user input that is input through a user interface for receiving a visual pattern, providing, through the user interface, a first visual object having a first visual pattern corresponding to the user input, wherein a first sound, which is substantially synchronized with a detection time point of the user input and has at least one characteristic identified based on the position of the user input defined in the user interface, is provided; and
based on identifying an event for providing a second visual pattern corresponding to the test sound, providing a second visual object having the second visual pattern corresponding to at least one characteristic of the first auditory pattern of the test sound.

27. The method of claim 26,
wherein providing the first visual object through the user interface comprises:
based on detecting at least a part of the user input associated with a first position and a second position of the user interface, providing at least a part of the first visual object associated with the first position, the second position, and at least one intermediate position between the first and second positions of the user interface.

28. The method of claim 27,
wherein based on at least a part of the user input being associated with the first position, a first portion of the first sound having at least one characteristic corresponding to the first position is provided;
based on at least a part of the user input being associated with each of the at least one intermediate position, at least one intermediate portion of the first sound having at least one characteristic corresponding to each intermediate position is provided;
and based on at least a part of the user input being associated with the second position, a second portion of the first sound having at least one characteristic corresponding to the second position is provided.

29. The method of claim 27,
wherein at least a part of the user input comprises an input for designating the first position and the second position, and/or an input for designating the first position, the at least one intermediate position, and the second position.

30. A method for auditory training by a system including a server and an electronic device, comprising:
providing, by a server including at least one first processor, instructions to the electronic device based on a connection to the server by the electronic device and/or a request to the server, wherein the server is configured to provide the instructions; and
executing, by the electronic device, the instructions, wherein the instructions, when executed based on at least a portion of at least one processor of the electronic device, cause the electronic device to perform at least one operation,
and the at least one operation comprises:
providing a test sound having a first auditory pattern for at least one characteristic of a sound;
based on a user input that is input through a user interface for receiving a visual pattern, providing, through the user interface, a first visual object having a first visual pattern corresponding to the user input, wherein a first sound, which is substantially synchronized with a detection time point of the user input and has at least one characteristic identified based on the position of the user input defined in the user interface, is provided; and
based on identifying an event for providing a second visual pattern corresponding to the test sound, providing a second visual object having the second visual pattern corresponding to at least one characteristic of the first auditory pattern of the test sound.

31. The method of claim 30,
wherein providing the first visual object through the user interface comprises:
based on detecting at least a part of the user input associated with a first position and a second position of the user interface, providing at least a part of the first visual object associated with the first position, the second position, and at least one intermediate position between the first and second positions of the user interface.

32. The method of claim 31,
wherein based on at least a part of the user input being associated with the first position, a first portion of the first sound having at least one characteristic corresponding to the first position is provided;
based on at least a part of the user input being associated with each of the at least one intermediate position, at least one intermediate portion of the first sound having at least one characteristic corresponding to each intermediate position is provided;
and based on at least a part of the user input being associated with the second position, a second portion of the first sound having at least one characteristic corresponding to the second position is provided.

33. The method of claim 31,
wherein at least a part of the user input comprises an input for designating the first position and the second position, and/or an input for designating the first position, the at least one intermediate position, and the second position.

34. A storage medium storing computer-readable instructions,
wherein the instructions, when executed by at least one processor of an electronic device, cause the electronic device to perform at least one operation,
and the at least one operation comprises:
providing a test sound having a first auditory pattern for at least one characteristic of a sound; based on a user input that is input through a user interface for receiving a visual pattern, providing, through the user interface, a first visual object having a first visual pattern corresponding to the user input, wherein a first sound, which is substantially synchronized with a detection time point of the user input and has at least one characteristic identified based on the position of the user input defined in the user interface, is provided; and
based on identifying an event for providing a second visual pattern corresponding to the test sound, providing a second visual object having the second visual pattern corresponding to at least one characteristic of the first auditory pattern of the test sound.

35. The storage medium of claim 32,
wherein providing the first visual object through the user interface comprises:
based on detecting at least a part of the user input associated with a first position and a second position of the user interface, providing at least a part of the first visual object associated with the first position, the second position, and at least one intermediate position between the first and second positions of the user interface.

36. The storage medium of claim 35,
wherein based on at least a part of the user input being associated with the first position, a first portion of the first sound having at least one characteristic corresponding to the first position is provided;
based on at least a part of the user input being associated with each of the at least one intermediate position, at least one intermediate portion of the first sound having at least one characteristic corresponding to each intermediate position is provided;
and based on at least a part of the user input being associated with the second position, a second portion of the first sound having at least one characteristic corresponding to the second position is provided.

37. The storage medium of claim 35,
wherein at least a part of the user input comprises an input for designating the first position and the second position, and/or an input for designating the first position, the at least one intermediate position, and the second position.

38. An electronic device, comprising:
at least one processor; and
a memory storing instructions,
wherein the instructions, when executed based on at least a portion of the at least one processor,
cause the electronic device to perform at least one operation,
and the at least one operation comprises:
providing a test sound having a first auditory pattern for at least one characteristic of a sound; based on a user input that is input through a user interface for receiving a visual pattern, providing, through the user interface, a first visual object having a first visual pattern corresponding to the user input, wherein a first sound, which is substantially synchronized with a detection time point of the user input and has at least one characteristic identified based on the position of the user input defined in the user interface, is provided; and
based on identifying an event for providing a second visual pattern corresponding to the test sound, providing a second visual object having the second visual pattern corresponding to at least one characteristic of the first auditory pattern of the test sound.

39. The electronic device of claim 38,
wherein providing the first visual object through the user interface comprises:
based on detecting at least a part of the user input associated with a first position and a second position of the user interface, providing at least a part of the first visual object associated with the first position, the second position, and at least one intermediate position between the first and second positions of the user interface.

40. The electronic device of claim 39,
wherein based on at least a part of the user input being associated with the first position, a first portion of the first sound having at least one characteristic corresponding to the first position is provided;
based on at least a part of the user input being associated with each of the at least one intermediate position, at least one intermediate portion of the first sound having at least one characteristic corresponding to each intermediate position is provided;
and based on at least a part of the user input being associated with the second position, a second portion of the first sound having at least one characteristic corresponding to the second position is provided.

41. The electronic device of claim 39,
wherein at least a part of the user input comprises an input for designating the first position and the second position, and/or an input for designating the first position, the at least one intermediate position, and the second position.
